# EUROPEAN PATENT SPECIFICATION

(11) **EP 3 708 197 B1**
(45) Date of publication and mention of the grant of the patent: **08.05.2024**
(21) Application number: 20169702.6
(22) Date of filing: 14.03.2014
(51) Int. Cl.: F04B 39/00, A61M 1/00

(54) **VACUUM CARTRIDGE WITH INTEGRATED VALVE**
VAKUUMKARTUSCHE MIT INTEGRIERTEM VENTIL
CARTOUCHE À VIDE À SOUPAPE INTÉGRÉE

(30) Priority: 15.03.2013 US 201361801626 P
(43) Date of publication of application: 16.09.2020
(62) Divisional of application: 14764093.2
(73) Proprietor: Solventum Intellectual Properties Company, Maplewood, MN 55144 (US)
(72) Inventor: CONNER, Ryan, San Antonio, TX 78265 (US); HU, Dean, San Antonio, TX 78265 (US); MUSANTE, Crystal, San Antonio, TX 78265 (US); WU, Kenneth, San Antonio, TX 78265 (US)
(74) Representative: Simmons & Simmons

(56) References cited:
- US-A- 5 071 409
- US-A1- 2009 005 747
- US-A1- 2010 228 205
- US-A1- 2011 295 220
- US-A1- 2012 209 225
- US-B1- 6 174 306

## Description

### BACKGROUND

The use of sub-atmospheric pressure to treat wounds can be traced back to ancient civilizations. For example, the ancient Chinese used "Cupping," a technique that creates reduced pressure environment by flaming a glass chamber to draw out bad humors from the body. Modem research has revealed that applying reduced pressure to a damaged tissue may have several beneficial effects: 1) a reduced pressure level may lead to retraction of the damaged tissue edges and thus may reduce the defect size and may expedite healing by facilitating wound contraction; 2) the reduced pressure may provide mechanical stimulation to the damaged tissue which may release growth factors at the wound bed to promote healing; 3) the reduced pressure may create suction in the damaged tissue cavity which may remove necrotic tissue from the damaged tissue cavity and may reduce bacterial load; 4) the application of reduced pressure may increase blood flow to the damaged tissue and, which may expedite healing; and 5) reduced pressure may remove granulation inhibiting metalloproteinase enzymes, which may enhance tissue remodeling and healing.

In light of the many benefits of reduced pressure tissue therapy, reduced-pressure wound treatment systems and methods are desirable.
US 6,174,306 concerns a device for vacuum-sealing an injury.
US 2009/0005747 A1 concerns a fluid collection and disposal system having interchangeable collection and other features and methods relating thereto.
US 2010/0228205 A1 concerns devices and methods to apply alternating level of reduced pressure to tissue.
US 2011/0295220 A1 concerns a reduced pressure delivery system having a manually-activated pump for providing treatment to low-severity wounds.
US 5,071,409 concerns a vacuum device particularly useful for draining wounds.
US 2012/0209225 A1 concerns a controlled negative-pressure apparatus and alarm mechanism.

### BRIEF SUMMARY

There is provided a system according to claim 1.

A selection of optional features is set out in the dependent claims.

### BRIEF DESCRIPTION OF THE DRAWINGS

A better understanding of various features and advantages of the embodiments described herein may be obtained by reference to the following detailed description that sets forth illustrative examples and the accompanying drawings of which:
Fig. 1 is a perspective view of one embodiment of the reduced pressure therapy device comprising a suction apparatus, an extension tube and a sealant layer.
Fig. 2 is a cut-away perspective view of one embodiment of a suction apparatus of Fig. 1 in a charged configuration.
Fig. 3 is a cut-away perspective view of one embodiment of a suction apparatus of Fig. 2 in a depleted configuration.
Fig. 4 is a perspective view of the embodiment of Figs. 2 and 3 with a reset tool.
Fig. 5 is perspective view of one embodiment of a suction apparatus.
Figs. 6A and 6B are posterior and anterior perspective component views of the embodiment from Fig. 5.
Fig. 7A depicts another embodiment of a reduced pressure therapy device comprising a clear collection chamber wherein the device is not charged (i.e., fully depleted); Fig. 7B depicts the device of Fig. 7A in a charged (i.e., fully charged) configuration; Figs. 7C and 7D or superior and side elevational views of the device in Figs. 7A and 7B in an activated and partially expended (e.g., partially depleted) state.
Fig. 8A is a superior elevational view of the suction chamber; Fig. 8B is a cross-sectional view of the distal end of the suction chamber.
Fig. 9A is a component view of a fitting assembly; Fig. 9B is a cross-sectional view of the fitting of the fitting assembly from Fig. 9A.
Fig. 10A is a schematic cut-away view of one embodiment of a connecting mechanism between a fitting and a suction chamber connector; Figs. 10B and 10C are cross-sectional views of the connecting mechanism from Fig. 10A.
Figs. 11A and 11B are posterior and anterior component views of one embodiment of a spring assembly, respectively.
Figs. 12A and 12B are posterior and anterior perspective component views, respectively, of one embodiment of a piston assembly and spring assembly. Fig. 12C is a front elevational view of the piston assembly.
Fig. 13 is a cross sectional view of one embodiment of a piston assembly coupled to a spring assembly.

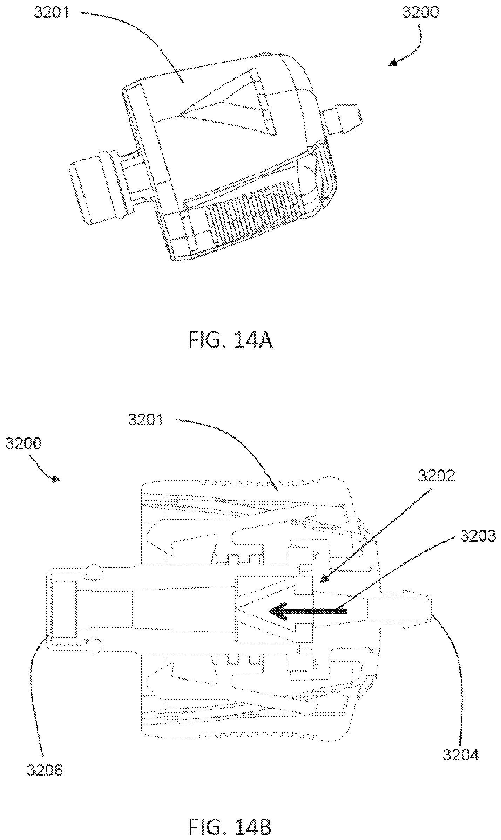

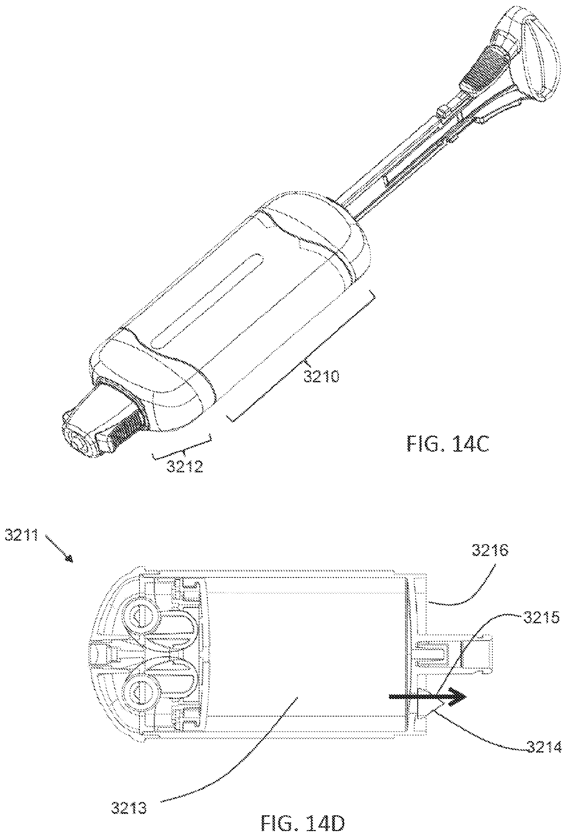

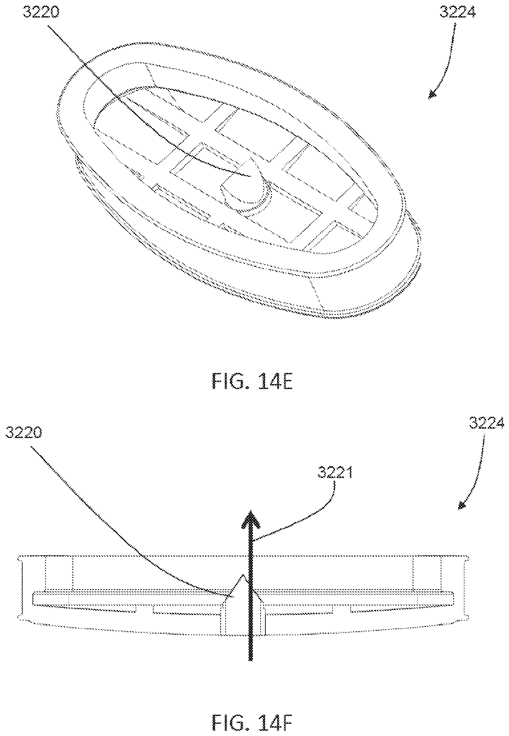

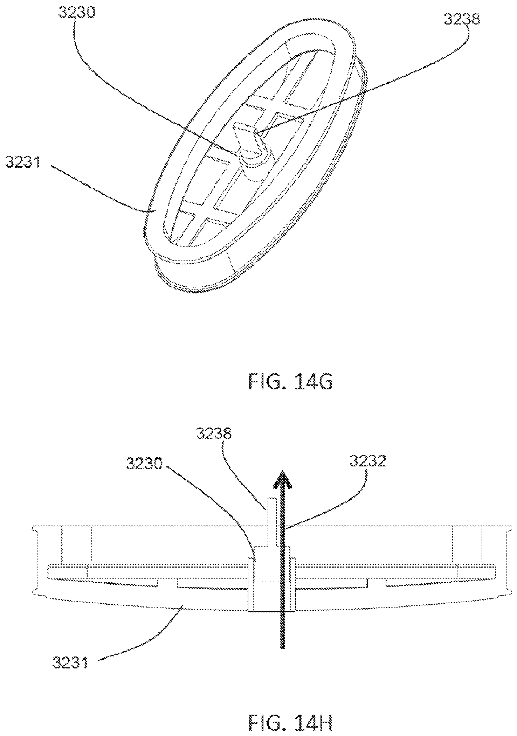

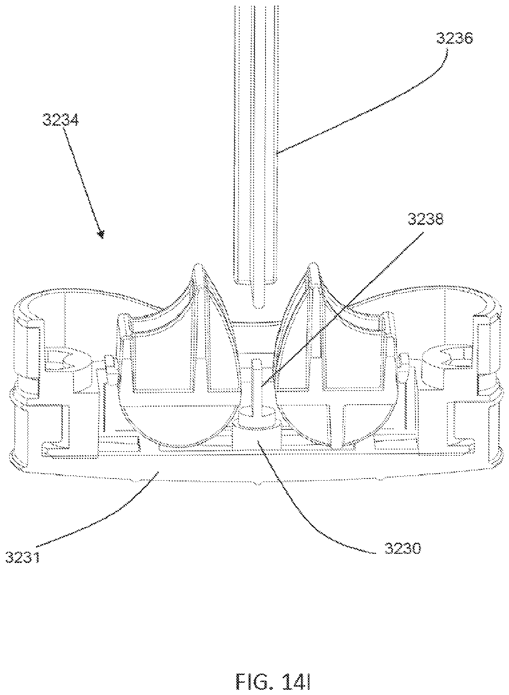

Figs. 14A and 14B depict one variation of a connector with an integrated valve. Fig. 14A is a perspective view of the connector and Fig. 14B is a cross-sectional view of the connector of Fig. 14A. Fig. 14C schematically depicts various locations for a second valve. Fig. 14D depicts one variation of a suction device with a valve located on the distal wall. Fig. 14E depicts a perspective view of one variation of a slidable seal having a valve across it. Fig. 14F depicts a cross-sectional view of the slidable seal of Fig. 14E. Fig. 14G depicts a perspective view of one variation of a slidable seal having a valve across it, corresponding to the claimed invention. Fig. 14H depicts a cross-sectional view of the slidable seal of Fig. 14G. Fig. 14I depicts a reset tool that may be used to with a piston assembly having the slidable seal of Figs. 14G and 14H.
Figs. 15A and 15B depict perspective distal views of one variation of a slidable seal comprising a one-way valve and a filter disposed over the valve. Fig. 15C depicts a close-up perspective view of a portion of the slidable seal of Figs. 15A and 15B. Fig. 15D depicts a perspective view of the slidable seal of Figs. 15A and 15B with a valve cover. Fig. 15E is a planar view of a valve cover. Fig. 15F is a side view of the slidable seal of Figs. 15A and 15B with a valve cover.
Fig. 16A is a schematic side view of a portion of a slidable seal with a valve cover in a first configuration. Fig. 16B is a schematic side view of a portion of a slidable seal with a valve cover in a second configuration.
Fig. 17A is a perspective view of absorbent pads that may be included in any of the suction devices described here. Fig. 17B is a perspective view of one variation of a restraint structure for the absorbent pads of Fig. 17A and Fig. 17C is a schematic depiction of the restraint structure of Fig. 17B. Fig. 17D is a schematic perspective view of the absorbent pads of Fig. 17A retained within the restraint structures of FIG. 17C. Fig. 17E is a schematic top view of the absorbent pads of Fig. 17A retained within the restraint structures of FIG. 17C.

While embodiments have been described and presented herein, those embodiments are provided by way of example only. Variations, changes and substitutions may be made without departing from the invention. It should be noted that various alternatives to the exemplary embodiments described herein may be employed in practicing the invention. For all of the embodiments described herein, the steps of the methods need not to be performed sequentially.

### DETAILED DESCRIPTION

Modern adaptations of techniques to provide reduced pressure to wounds have been developed recently. There are several commercially available models of these types of reduced pressure dressing systems. These devices may comprise an interface layer that is placed into the wound, an occlusive layer that creates a seal around the wound, connection tubing that is in fluid communication with the interface layer and the wound, a separate exudates collection canister, and an electric pump that provides a source of vacuum. However, the electric pumps are bulky and heavy thereby reducing patients' mobility especially during prolonged treatment periods. These electrical pumps, in operation, can be noisy and conspicuous. Further, the placement of the interface layer, the occlusive layer, and the connection tubing is labor intensive and time consuming increasing patient dependence on health care professionals and further leading to higher health care costs. These systems typically have non-disposable pumps and systemic components that require significant maintenance and servicing and that carry the risk of spreading contamination and infection. Although these systems can be used to treat smaller wounds, they are designed to treat large wounds and are not usually used to treat smaller wounds. Since current systems depend on electrical power for their operation, they further constrain patient movement to areas having electricity or rely on limited battery power where no electricity is available.

Described herein are devices configured to apply reduced air pressure (i.e., a vacuum) to a treatment site, such as a damaged tissue cavity or other type of wound. In some embodiments, the device may also be used to apply reduced pressure to otherwise undamaged tissue. In one embodiment, the tissue therapy device may comprise a sealant layer and a suction apparatus. The sealant layer may be used to create a seal around an area of tissue requiring therapy. The suction apparatus fluidly communicates with the sealed enclosure formed by the sealant layer and reduces pressure within the enclosure adjacent to the damaged tissue. In some embodiments, the suction apparatus may be non-electrically powered. For example, the suction apparatus may be configured to self-generate reduced pressure, i.e., without requiring a separate power or vacuum source. A reduced pressure therapy device comprising a self-generating reduced pressure mechanism may provide a patient with freedom and mobility without concerns of running out of battery power or having access to an electrical outlet or vacuum generator. The sealant layer and the suction apparatus may be used to form a closed reduced pressure system to resist the backflow of gas into the system.

The reduced pressure may be self-generated by expanding the volume of air initially located in the sealed enclosure and/or suction apparatus from a smaller volume of the enclosure to a larger volume shared between the sealed enclosure and the suction apparatus. Upon expansion of the air within the sealed enclosure, the density of the air molecules is decreased and the pressure within the sealed enclosure is reduced to a sub-atmospheric level.

In one embodiment the tissue therapy device comprises a contact layer matrix that is placed into or over the wound bed or other tissue defect. In some embodiments, the contact layer matrix may be used to distribute the reduced pressure more evenly through the wound bed, and may also provide a scaffold or contact surface which promotes healing. In another embodiment, the damaged tissue cavity, packed with the contact layer matrix, is then placed under a sealant layer to produce a sealed enclosure containing the contact layer and the wound bed. Fluid communication to the interior of enclosure is provided by an attachment port of the sealant layer.

In some embodiments, the attachment port may comprise a collar with an inlet opening, a soft elastomeric body, and an outlet port. In some examples, the collar may comprise a rigid or flexible material, and the collar may be oriented at any of a variety of angles with respect to the sealant layer, including a perpendicular angle. The outlet port of the attachment port may also be flexible or rigid, and may be oriented at any of a variety of angles with respect to the sealant layer or collar. In some examples, the outlet port may be oriented generally parallel to the plane of the sealant layer, or even below the parallel plane of the sealant layer, depending upon the height of the collar, but in other examples, the outlet port may be bent or angle above the plane of the sealant layer. The various components of the attachment port may or may not be directly connected to one another, and the inlet and the outlet may have some degree of freedom of movement relative to one another.

In some embodiments of the device, the device may comprise a sealant layer made of a hydrocolloid material or any other material known to those skilled in the art. The hydrocolloid sealant layer may be semi-porous and breathable to absorb moisture from the wound while protecting the skin. In addition, the hydrocolloid sealant layer is typically thicker than other materials such as acrylic adhesives to allow for easier placement with less folding and wrinkling and to seal potential fluid leak paths.

In one embodiment of the device disclosed herein, the attachment port is directly mounted to a distal portion of the suction apparatus. In other embodiments the attachment port is connected to the suction apparatus via an extension tube. In some embodiments, the extension tube may be adapted to mitigate entanglement. The suction apparatus and the extension tubing may have similar fittings and release buttons to prevent accidental disconnection. In embodiments in which extension tubing is used, the distal end of the extension tubing is connected to the distal end of the suction apparatus with similar fitting.

Some embodiments of the device disclosed herein comprise a pressure gauge integrated into the attachment port or another component. The mounting of the pressure gauge into the attachment port enables accurate measurement of pressure level within the enclosure adjacent to the wound and formed by the sealant layer. The pressure gauge described herein may less susceptible to incorrect pressure readings that are typically caused by clots in the tubing connecting the reduced pressure source to the wound.

In some embodiments of the reduced pressure system disclosed herein, the suction apparatus reduces the air pressure within the enclosure adjacent to the damaged tissue by forcefully expanding the volume of air within the enclosure without changing the external dimensions of the reduced pressure generating unit. In other embodiments, the tissue therapy device may self-regulate the pressure to a substantially constant level.

In one embodiment, the suction apparatus comprises a chamber, a slidable seal, a valve, and an activation system. The suction cartridge may comprise a release button and an activation button in a distal portion. The activation button may be connected to a sliding blade valve which prevents fluid communication from the enclosed area adjacent to the wound to the chamber when in the "off' position. When the activation button is depressed, the sliding blade valve may switch to an "on" position to permit fluid communication from the enclosure to the chamber. The activation button may be spring loaded to be biased to the "off' position but once it is depressed, a spring-loaded latch may engage to remain in the "on" position. The release button may be adapted and configured to allow detachment of any article (e.g., extension tubing or sealant layer attachment port) from the suction apparatus and to terminate fluid communication between the suction chamber and the enclosed area. The release button may engage the interlock segment to pull the latch away from the activation button. If the activation button is in the "on" position, it will revert back to the "off' position by virtue of the spring loading.

In one embodiment of the reduced pressure system, the suction chamber comprises an ellipsoidal cylinder having a slidable seal concentrically disposed therein. The chamber has a variable effective volume defined by the distance between the distal end of the chamber, which is located adjacent to the opening connected to the sliding blade valve and a current position of the slidable seal. In the fully charged state, the seal is closest to the distal end of the suction cartridge, and the effective volume of the chamber is zero or nearly zero. The slidable seal may be connected to one or a series of springs which may be used to bias the seal towards an activated state where the effective volume of the chamber is the maximum. The springs may have any of a variety of configurations, including ribbon springs. The ribbon spring may be a substantially constant force spring or a variable force spring. In some examples, a combination of spring types may be used. In still other examples, a single ribbon may be configured with a coil at each end and attached to a slidable seal at a middle region of the single ribbon. In one embodiment of the device, the spring(s) may exert a force of less than 0.5 pounds. In other embodiments of the present invention the constant force spring(s) may exert a force of less than 1 pound. In some embodiments of the reduced pressure system the constant force spring(s) may exert a force of less than 5 pounds. In other embodiments of the device disclosed herein the substantially constant force spring(s) may exert a force of less than 20 pounds. In other examples, the force per square inch exerted across the collection volume of the device may be in the range of about 0.1 psi to about 50 psi, in some examples about 0.5 to about 20 psi, and in other examples about 1.5 psi to about 5 psi. This pressure may be exerted by a single force member or may be the aggregate pressure from two or more force members. The force or pressure may be selected based on the type, size, location, or another suitable characteristic of the wound being treated.

In some embodiments of the tissue therapy system the suction cartridge is fabricated from a rigid polymer adapted to maintain the external shape of the suction chamber shape under reduced pressure. The suction chamber can be made of any suitable polymer such as, but not limited to polycarbonate, co-polyester, polyethylene, polypropylene, acrylic, ABS, glass, medical-grade polymers, or a combination thereof.

In other embodiments of the reduced pressure system, the slidable seal is fabricated from a material adapted to create an airtight separation between the portion of the suction apparatus below it and the remainder of the suction apparatus. The material may be elastomeric or non-elastomeric. The slidable seal can be made of materials such as: silicone, fluorosilicone, nitrile, natural rubber, thermoplastic elastomer, thermoplastic urethane, butyl, polyolefin, polyurethane, styrene, polytetrafluoroethylene, any other suitable material, or a combination thereof.

In some embodiments of the tissue therapy system, the suction cartridge may be coated using a friction mitigating lubricant to reduce movement of the slidable seal due to friction within the suction chamber and to reduce the likelihood of the seal sticking after being in a static position for prolonged periods. The lubricant coating material may be polydimethysiloxane, perfluoropolyether, mineral spirits, synthetic oils, polyxylene, any other suitable lubrication polymer or material, or any combination thereof.

In one embodiment of the reduced pressure system disclosed herein the suction apparatus springs are placed in a high potential energy extended state prior to activation. In other embodiments of the device, prior to activation, the sliding blade valve is closed and the chamber is completely sealed. In such embodiments, the springs are prevented from retracting because the extremely small volume of air in the chamber resists the expansion that would be caused by the constant force springs' retraction of the slidable seal. The device is ready to be activated once the wound bed is sealed with the sealant layer, and the sealant layer is connected to the suction cartridge either directly or via an extension tube.

When the tissue therapy system disclosed herein is activated, fluid communication is established between the suction chamber and the sealed wound enclosure. Since there is a finite amount of air within the enclosure (which is initially at atmospheric pressure), upon activation, the constant force springs will retract the slidable seal and expand the effective volume of the suction chamber. As known by the ideal gas law, as a volume of air expands adiabatically, the pressure of the air will be reduced, and subject the sealed wound enclosure to reduced pressure.

In some embodiments, the tissue therapy system may be used to maintain a substantially constant level of reduced pressure despite the presence of exudates and air leaked into the system. The slidable seal is a mechanical system wherein the seal position is adapted and configured to be in equilibrium based on the traction of the substantially constant force springs, other traction elements in the system, and/or the pressure differential across the chamber seal. Other traction elements in the system may include frictional forces (i.e. static and/or kinetic frictional forces). If the reduced pressure were to recover towards atmospheric within the chamber, the pull of the springs would be greater than the pull due to the pressure differential. This, in turn, will force the springs to retract and cause a simultaneous increase in the volume of the chamber. This increase in volume will result in a reduction of pressure away from atmospheric pressure within the chamber, until a new equilibrium is reached where the pressure differential and the substantially constant spring force reach a new equilibrium. In some embodiments, the walls of the suction chamber are straight thereby ensuring that the level of reduced pressure stays constant regardless of the actual position of the seal within the chamber.

In some embodiments, the suction apparatus may be configured to generate a reduced pressure which may be generally characterized by the absolute pressure level and/or by a pressure level reduction relative to the atmospheric pressure. In some embodiments, the device is configured to generate a level of reduced pressure between about 0 and about 760 mmHg. In some embodiments, the generated amount of reduced pressure in the enclosure formed by the sealant layer and treatment site is more than about 10 mmHg, about 20 mmHg, about 50 mmHg, about 80 mmHg, about 100 mmHg, about 150 mmHg, about 200 mmHg, about 500 mmHg, about 700 mmHg, or even about 750 mmHg or more. The device may generate an absolute reduced pressure underneath the sealant layer where the reduced pressure is anywhere between about 0 and about 760 mmHg. In some embodiments, the generated level of reduced pressure in the enclosure formed by the sealant layer is less than about 700 mmHg, sometimes less than about 600 mmHg, other times less than about 400 mmHg, or even less than about 250 mmHg, about 125 mmHg, about 75 mmHg, about 50 mmHg, less than about 25 mmHg, or less than about 10 mmHg. In some embodiments, the sealant layer generally follows the perimeter of the area of tissue being treated. The tissue therapy devices may have different collection chamber sizes which allow for treatment of larger, more exudative wounds while maintaining the smallest configuration possible for enhanced usage comfort. This may be particularly advantageous for small wounds or treatment sites, as a smaller reduced pressure source can be partially or fully integrated into the dressing or sealant layer. In some embodiments, the cavity of the suction apparatus is about 50 cc or less in volume, while in other embodiments, the cavity may be about 100 cc in volume. In other embodiments, the collection chamber is less than about 150 cc in volume. In some embodiments, the collection chamber is less than about 200 cc in volume. In other embodiments, the collection chamber is smaller than about 300 cc in volume. In some embodiments, the collection chamber is less than about 500 cc in volume. In other embodiments, the collection chamber is less than about 1000 cc in volume. In other embodiments, the cavity of the suction apparatus may be at least about 50 cc, about 100 cc, about 150 cc, about 200 cc, about 300 cc, about 500 cc or about 1000 cc or more.

In certain embodiments, the device comprises an elongated rigid member that fits into an opening the proximal end of the suction apparatus and serves as a lever that charges the constant force springs with potential energy by pressing the springs towards the device's distal end until the latch, embedded within said lever, locks into place. In some embodiments, the elongated member is integrated into the suction apparatus body and serves as a cap to the suction apparatus. In some embodiments, the elongated lever enables safe storage of the suction apparatus as it prevents the springs from retracting due to micro-leaks that may cause the springs to lose the energy stored in them. In other embodiments, it enables recharging of the spring mechanism when accidental discharge occurs or an undetected leak is present while the device is in use.

In some embodiments, the suction apparatus comprises an elongated rigid member adapted and configured to be inserted into a mating opening in the proximal end of the suction generating unit. The elongated rigid member contacts the rigid portion of the chamber seal and thus can be used to mechanically push the seal down the chamber against the constant force springs thereby imparting potential energy into the constant force springs. This pushing motion is completed with the suction cartridge disconnected from the extension tubing or attachment port, and with the activation button and the sliding blade valve in the off position. Once the slidable seal reaches a point close to maximum spring extension, a latch tab on the elongate rigid member will engage a slot in the suction apparatus body and prevent spring retraction. At this point, the sliding blade valve should be closed by depressing the release button thereby sealing the chamber. The elongate member can then be removed by pressing the latch tab leaving the suction apparatus ready for activation.

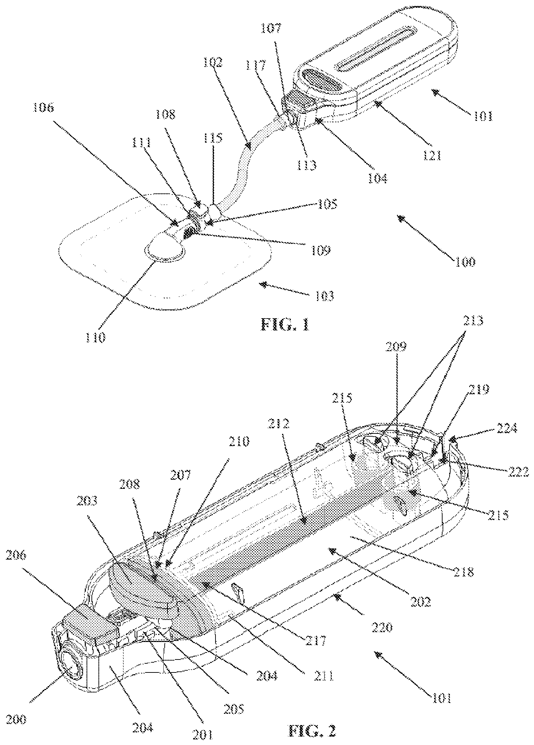

Fig. 1 illustrates one embodiment of a reduced pressure therapy device 100, comprising a suction apparatus 101, an extension tube 102, and a sealant layer 103. The sealant layer 103 may further comprise an integrated attachment port 106 configured to connect the sealant layer 103 to the extension tube 102 and/or directly to the suction apparatus 101. In some embodiments, the connector of the extension tube 102 or suction apparatus 101 may be configured to rotate about an axis of attachment port 106. In some embodiments, the attachment port 106 may be configured to rotate around its base 110 and/or to bend toward and/or away from the sealant layer 103. For example, the attachment port 106 may be configured to freely rotate about 360 degrees or more, or to provide a limited rotation range less than about 360 degrees, including but not limited to about 315 degrees, about 270 degrees, about 225 degrees, about 180 degrees, about 135 degrees, about 90 degrees, or about 45 degrees, for example. In other embodiments, the tubing connector and/or the connector interface of the attachment may be configured to rotate with respect to the longitudinal lumen axis. The attachment port 106 may have a fixed orientation that is generally parallel to the plane of the sealant layer, but in other configurations, may be angled below the parallel plane or above the parallel plane. In still other examples, the attachment port 106 may be configured to bend or pivot relative to the sealant layer 103. The range of bending or pivoting may be from about 0 degrees to about 45 degrees or about 90 degrees, from about 0 degrees to 135 degrees or about 180 degrees, or from about -15 degrees or about -30 degrees to about 45 degrees, about 90 degrees, about 135 degrees, about 180 degrees, 195 degrees or about 210 degrees. In certain embodiments, the attachment port 106 may be configured to rotate and pivot.

The extension tube may be coupled to the attachment port by any of a variety of mechanisms. For example, the attachment port may comprise a resistance or interference fitting which may be inserted into the lumen of the extension tube. The resistance fitting may comprise one or more flanges configured to resist decoupling of extension tube. In other examples, the extension tube may be inserted into the lumen or opening of the attachment port, and the attachment port may comprise a push-in fitting, such as a John Guest fitting (Middlesex, UK). In other embodiments, connectors on both components may be used, including threaded or mechanical interlocking fits. The connectors may be configured to facilitate both coupling and decoupling of the components.

In the example depicted in Fig. 1, one end of the extension tube 102 comprises a port connector 105 configured to couple to a connector interface 111 of the attachment port, and the other end may comprise a suction apparatus connector 107 configured to couple to a connector interface 113 of the suction apparatus 101. In the depicted embodiment, the connector interface 111 of the attachment port 106 and the suction apparatus connector 107 of the extension tube 102 may comprise male-type connectors, while the connector interface 113 of the suction apparatus 101 and the port connector 105 of the extension tube 102 may comprise female-type connectors. The particular male-female configuration described above is merely exemplary, and in other embodiments, the male/female configuration may be reversed, any other type of complementary interface may be used, including interfaces which are non-directional and permit the connector of the extension tube 102 in any direction. These or other complementary configurations may be used to permit both the direct connection of the suction apparatus 101 and the sealant layer 103, as well as the optional use of the extension tube 102. In some embodiments, the extension tube(s) and/or the extension tube connector(s) may be configured so that multiple extension tubes may also be joined together, either in a specific order or in any order. The extension tube may also comprise one or more stress-relief or anti-kink structures, e.g. a helical winding or other tubular support, which may resist pinching or other deformations of the tube. In Fig. 1, for example, the port connector 105 and the suction apparatus connector 107 of the extension tube 102 comprises a flared openings 115 and 117, respectively, which permit at least some deflection of the tube 102 relative to the connectors 105 and 107 while distributing the bending stress along the length of the flared opening 115 and 117 to resist pinching. In other embodiments, the stress relief structures of the connectors comprise one or more bendable or deformable projections, which may or may not be flared.

One or more connectors of the extension tube may also comprise a locking mechanism to facilitate decoupling and/or attachment of the extension tube. In some examples, a locking mechanism may resist inadvertent decoupling from the sealant layer and/or suction apparatus. In the example depicted in FIG. 1, the port connector 105 of the extension tube 102 comprises a locking mechanism with a connector release button 108 configured to resist decoupling until the button 108 is pressed. The connector release button 108 may be coupled to a movable structure that forms an interlocking or resistance fit with a complementary structure or surface on the attachment port 106. In some embodiments, the connector release button 108 may be spring loaded or otherwise biased, and may or may not provide additional sealing and/or locking force between the connector 105 and the attachment port 106. In other variations, other locking interfaces, including sliders, levers or knobs, may be used. The attachment port 106 may comprise one or more gripping materials or textured gripping surfaces 109. The gripping surface 109 on the exterior of the attachment port 106 may facilitate manual connection and disconnection of the connectors on the extension tube 102 or the suction apparatus 101. The grip surface 109 may comprise one or more flanges or ridges, for example, and/or a high traction material such as rubber or a block copolymer with polystyrene and polybutadiene regions, e.g., KRATON^{®} polymers by Kraton Polymers, LLC (Houston, Texas). Gripping materials or structures may also be provided on the connectors 105 and 107 and/or the suction apparatus 101. In Fig. 1, for example, the suction apparatus 101 comprises a nosepiece 104 having a reduced width relative to the body 121 of the suction apparatus 101. The nosepiece 104 may facilitate gripping of the suction apparatus 101 when detaching or pulling it apart the extension tube 102 or attachment port 106.

In some embodiments, the suction apparatus may comprise a rigid polymer configured to generally maintain its shape under reduced pressure. The suction apparatus can be made of any suitable polymer such as polycarbonate, co-polyester, polyethylene, polypropylene, acrylic, ABS, glass, or any other polymer known to those skilled in the art.

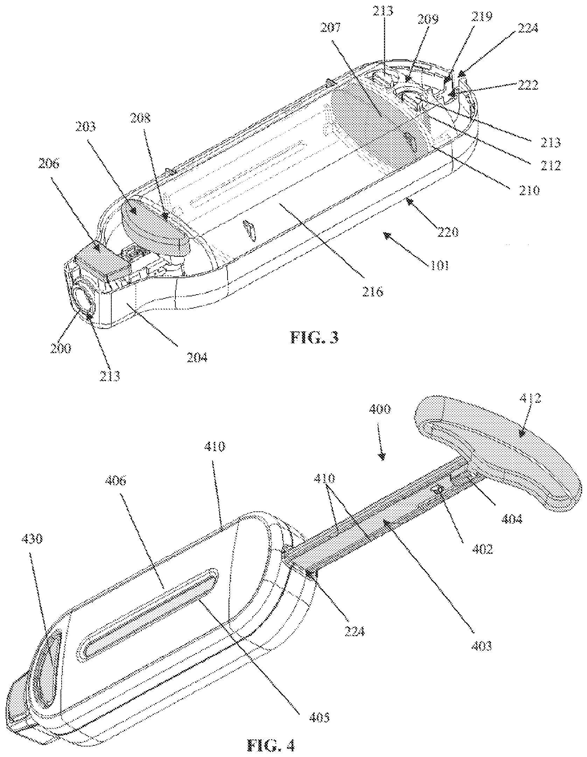

Figs. 2 and 3 are detailed views of one embodiment of the suction apparatus 101 in Fig. 1. The connector interface 113 may comprise a connector 200 which may be coupled to the connector 107 at the proximal end of the extension tube 102, and/or the connector interface 111 of the attachment port 106 as depicted in Fig. 1. The suction apparatus 101 comprises a slidable seal 207 located inside a suction chamber 202. Fig. 2 depicts the slidable seal 207 in a distal position near the distal end 208 of the suction chamber 202, and Fig. 3 depicts the seal 207 in a proximal position near the proximal end 209 of the suction chamber 202. The slidable seal 207 may be mounted on a seal mount 210 and is configured to traverse between the distal end 208 and proximal end 209 of the chamber 202 while maintaining a substantial airtight seal. The suction chamber 202 may be also be characterized by the portions of the chamber 202 separated by the seal 207. For example, the suction chamber 202 may comprise a collection chamber 216 located between the distal end 208 of the chamber 202 and the seal 207, and a working chamber 218 between the proximal end 209 of the suction chamber 202 and the seal 207. The collection chamber 216 may be configured to generate a reduced pressure and is in fluid communication with the connector 200 to provide reduced pressure under the sealant layer 103. In the particular embodiment depicted in Figs. 2 and 3, the collection of materials suctioned from a wound and the generation of reduced pressure both occur in the collection chamber 216, but in other embodiments, the collection chamber and reduced pressure generating chamber may be different structures.

The working chamber 218 of the suction apparatus 101 may contain one or more force or bias members, and may also provide access to the seal 207 to permit charging or resetting of the force or bias members. A portion of the force or bias members may be attached or fixed to a portion of working chamber 218, while another portion is attached to the seal 207. In the particular embodiment depicted in Fig. 2, the force member comprises two constant force springs 212 with proximal ends 215 mounted in the working chamber 218 using posts or pins 213, while their distal ends 217 are attached a seal mount 210 that is coupled to the seal 207. In some embodiments, the seal 207 and the seal mount 210 may be integrally formed. The slidable seal 207 may mounted on a seal mount 210 by methods such as injection over-mold, adhesive bonding, or mechanical bonding, or by a mechanical resistance or interlocking fit. In other embodiments, the force members may be directly coupled to the seal 207. The functionality and structure of the seal 207 is described in greater detail below.

The volumes of the collection chamber 216 and the working chamber 218 may vary, depending upon the position of the seal 207. In Fig. 2, where the seal 207 is in an extended position and in a charged configuration, the effective volume of the collection chamber 216 may be about zero or close to zero. In Fig. 3, wherein the seal 207 is in a retracted position, the effective volume of the collection chamber 216 may be at or near the volume of the suction chamber 202, notwithstanding the volume taken up by the seal 207, seal mount 210 and/or the bias members. In other examples, the volume of the collection chamber may be generally based upon the equilibration of the force generated by the bias members and the counteracting force resulting from the reduced pressure generated in the collection chamber 216. The volume of the working chamber 218 may be inversely related to the volume of the collection chamber 216. In some instances, the maximum volume of the working chamber 218 may be less than the volume of the suction chamber 202, which may result from volume displacement by the seal 207 or seal mount 210, and/or by other structures located within the working chamber 218 or structures which limit the movement range of the working chamber 218.

Access to the seal 207 may be achieved through the access opening 224 located about the distal end 209 of the housing 220. As the slidable seal 207 traverses from the extended position as depicted in Fig. 2 to the retracted position as depicted in Fig. 3, the interior volume of the collection chamber 216 increases from about zero to about the maximum volume provided in the fully retracted position, the suction apparatus 101 comprises a collection chamber 216 with the maximum effective collecting volume. When the collection chamber 216 is in airtight fluid communication with a sealed wound enclosure and a good dressing seal is obtained within the wound enclosure, expansion of the volume of the collection chamber 216 will reduce the pressure level in the sealed wound enclosure to a point where an equilibrium between the restoring force applied on the slidable seal 207 by the constant force springs 212 and the pressure differential across the slidable seal 207 is reached.

Some embodiments of the suction apparatus 101 may further comprise a valve 201 which may be configured to selectively permit fluid communication between the connector 200 to a collection chamber 216. The valve 201 may have any of a variety of configurations, including a rotating cylinder valve or a blade valve, for example. The valve may also be a multi-directional valve, a bi-directional valve or a one-way valve. The configuration of the valve 201 may be controlled by an activation button 203 or other type of actuator (e.g. a knob, switch, lever or slider). In some embodiments, the activation button 203 may comprise a first configuration where the chamber valve 201 closes or blocks fluid communication between the collection chamber 216 and the connector 200, and a second position where the valve 201 is open or allows passage of air and/or exudates to flow from the connector to the collection chamber 216. In some further embodiments, some valves may have additional configurations to selectively permit infusion of materials into the suction apparatus 101 and/or into the sealant layer, and/or configurations to selectively permit removal of air and/or materials from the collection chamber.

In further embodiments, a spring mechanism 204 may bias the valve 201 or its actuator to a closed or open position. For example, the spring mechanism 204 may be configured to bias the valve 201 to a closed position which blocks fluid communication between connector 200 and the collection chamber 216. When the valve 201 is actuated to open the fluid communication, a latch mechanism 205 or other type of locking mechanism may be used to engage the valve 201 and prevent the spring mechanism 204 from closing the valve 201. The locking mechanism may also comprise a release mechanism configured to permit selective disconnection or separation of an extension tube or sealant layer. For example, the connector 200 may be configured to prevent or resist disconnection of any components connected to the suction apparatus 101 through the connector 200 until a release button 206 or other actuator is depressed or manipulated. The release mechanism may comprise one or more displaceable or movable resistance or interlocking structures, for example. In other embodiments, the lock and/or release mechanism may be located on the extension tube or the attachment port of the sealant layer.

In some embodiments, the release button 206 may comprise a mechanism to control the valve 201. For example, the release button 206 may be configured to disengage the latch 205 from the valve 201, which permits the spring mechanism 204 to reposition the valve 201 to the closed position blocks permit fluid communication between the connector 200 and the collection chamber 216. In other embodiments, the release button 206 may be configured to control a second valve in the fluid communication pathway.

In some embodiments, the suction apparatus 101 may comprise a suction chamber 202 with a non-circular cross-sectional shape, with respect to a transverse or perpendicular plane to the movement axis of the seal 207. The non-circular cross-sectional shape may include but is not limited to a generally rectangular or generally ellipsoidal shape, for example. The suction apparatus 101 may comprise a first transverse dimension that is greater than a second transverse dimension, wherein each transverse dimension is transverse to the movement axis of the slidable seal 407. In some embodiments, the ratio of the first transverse dimension and the second transverse dimension is at least about 1.5, sometimes at least about 2, and other times at least about 3, or about 5 or more.

To prepare the suction apparatus 101 for generating a reduced pressure level in the sealed wound enclosure, the device is charged, i.e., the slidable seal 207 and the substantially constant force springs 212 may be placed in a distal or extended position within suction chamber 202. Charging of suction apparatus 101 may be performed using a push mechanism or tool inserted through an opening 224 configured to provide access to the seal 207 or seal mount 210. Examples of a push mechanism including the charging or reset tool 400 depicted in Fig. 4, which is described in greater detail below. Although a reset tool is used to place the suction apparatus into a charged state (e.g., into the fully charged or a partially charged state) after the suction apparatus has been used, the reset tool may also be used to initially charge the suction apparatus prior to first use. In some variations, the suction apparatus may be placed in a fully charged state during manufacturing where the reset tool is inserted through the opening and contacting the seal or seal mount. Prior to its first use, the charged suction apparatus may be activated by removing the reset tool. Referring back to Fig. 2, the slidable seal 207 is placed at an extended position, with the constant force springs 212 also in an extended state and charged with potential energy. In some embodiments, when the suction apparatus 101 is charged, the blade valve 201 is closed to seal the collection chamber 216. In these embodiments, retraction of the seal 207 by the constant force springs 212 is resisted or prevented because the small volume of air in the collection chamber 216 resists the expansion that would be caused by the retraction of the constant force springs 212. The suction apparatus 101 may comprise a locking mechanism to keep the slidable seal 207 in the fully charged position. In some embodiments, the charging mechanism or tool may also be used to keep the slidable seal 207 in position and resist retraction by the constant force springs 212

Once the wound bed is sealed with a sealant layer and the charged therapy device is connected to the suction apparatus, the charged therapy device may be activated to generate reduced pressure in the wound bed. In some embodiments, a user of the therapy device described herein may activate the therapy device by pressing down the activation button 203. In some examples, prior to activation, the activation button 203 may be biased to the "off' position. Pressing down or otherwise manipulating the activation button causes the valve 201 to open fluid communication between the collection chamber 216 and the sealed enclosure. Once the activation button 203 is pressed down, a spring-loaded latch on the interlock piece may engage to keep the activation button 203 in the "on" position.

When the reduce pressure therapy device is activated, fluid communication is established between the sealed wound enclosure and the collection chamber 216. If a sufficient dressing seal is obtained within the sealed enclosure, there should be a finite amount of air and/or exudate within the sealed enclosure which is initially at atmospheric pressure. Upon activation of the suction apparatus 101, the charged constant force springs 212 that are will then retract the slidable seal 207 and expand the volume of the collection chamber 216. Movement of the slidable seal 207 will stop at an equilibrium position where the traction force of constant force springs 212 is equal to the pressure differential across the slidable seal 207.

As the collection chamber is filled with exudates and/or air from potential air leakage into the sealed wound enclosure or other location in the system, the slidable seal 207 will retract towards the proximal end 209 of the suction chamber 202 until the constant force springs 212 reach a retracted position, as depicted in Fig. 3. Further retraction may be stopped either by a limit structure (if any) in the suction chamber 202, or as a result of the decreasing counterbalancing force as the reduced pressure collection chamber 216 returns to atmospheric pressure from increases in the joint volume shared by the wound enclosure and the collection chamber 216. The therapy device may then be removed from the treatment site, or the suction apparatus 101 may be disconnected from the sealant layer 103. As mentioned previously, disconnection may be achieved by pressing or actuating the release button 206. Once the release button 206 is pressed down or actuated, the blade chamber valve 201 will be engaged in its "off' position which will terminate or block any fluid communication between the sealed wound enclosure and the collection chamber 216. Also, the spring-loaded latch 205 on the interlock piece that forces or maintains the activation button 203 in the "on" position will be pulled away or otherwise manipulated to permit the activation button 203 will revert to its "off' position.

As depicted in Fig. 4, the tissue therapy system comprises a charging or reset tool or rod 400 which is inserted into the suction apparatus 101. The rod 400 may be pushed through an opening 224 of the housing 220 to push the slidable seal towards the distal end 208 of the suction chamber 202 and to charge the constant force springs with potential energy. In some embodiments, the suction apparatus 101 may be configured so that the reset tool 400 contacts or engages the seal mount (210 in Fig. 2) at or adjacent to where the constant force springs 212 are coupled to the seal mount 210. In other embodiments, the suction apparatus 101 may be configured such that the reset tool 400 directly pushes against the springs 212, in addition to or in lieu of pushing against the seal mount 210. In some embodiments, once the slidable seal is moved to the fully charged configuration, a locking structure or latch 402 located on the shaft 403 of the reset tool 400 may engage a complementary structure (e.g. slot 219 in Fig. 3) of the housing 220. Thus, the reset tool 400 may be used to lock the seal into its fully charged configuration and resist the constant force springs from retracting and losing its potential energy. The reset tool 400 may also comprise a handle 412 to facilitate gripping and use of the tool 400.

In other examples, the charging mechanism may be used without removing the reset tool from the device. In these embodiments, as the seal retracts, the reset tool will extend out of the accessing opening of the housing. In still other examples, a charging mechanism other than a linear push-based mechanism may be used, including but not limited to one or more rotatable knobs that may be used to unwind and extend the substantially constant force springs or other bias members to charge the device. In some other examples, where the force required to overcome the springs and charge the device may be excessive, the reset tool may be threaded and the reset tool opening may be configured with a screw driver coupled to a handle that may provide a mechanical advantage to a user charging or resetting the device. In still other examples, embodiments comprising a rotatable knob may comprise a slide-out handle, a swing out handle or an attachable handle to provide the user with greater torque when winding the knob.

Referring back to Figs. 2 and 3, the access opening 224 may be configured to restrict or limit pivoting or angulation of the reset tool 400 during insertion. The housing 220 may also comprise guides 222 that may further restrict or limit the motion of the reset tool 400 during insertion. The reset tool 400 may also comprise guide structures. Fig. 4, for example, depicts the reset tool 400 with ridges or raised edges 410 which may facilitate tracking of the shaft 403 along the constant force springs 212 as the springs 212 are extended. The distal end of the reset tool 400 and/or the seal mount 210 may be configured with complementary interfaces to resist decoupling as force is being applied using the reset tool 400.

In some embodiments, the charging or resetting procedure described above may be performed when the suction apparatus disconnected from any other components, e.g., extension tubing, attachment port or sealant layer. After charging the suction apparatus, the suction apparatus is attached to a sealant layer, directly or through extension tubing, the reset tool is removed, and the activation button on the suction apparatus is pressed to apply a reduced pressure within the sealed wound enclosure created by the sealant layer. In other embodiments, this charging process is completed with the activation button in the "off' position. Such design may prevent elevated pressure from being applied onto the damaged tissue inadvertently. A one-way valve in communication with the collection chamber may also be provided to expel air from the collection chamber during the charging procedure. Referring still to Figs. 3 and 4, in some embodiments, once the suction apparatus 101 is fully charged, a latch tab 404 or other actuator on the shaft 403 of the reset tool 400 can be pressed or manipulated to disengage the latch 402 from the interlocking slot 215, thereby allowing the reset tool 400 to be withdrawn from the suction apparatus 101. In some embodiments, the reset tool 400 may be left in the suction apparatus to ensure safe storage of the suction apparatus since it prevents the constant force springs from retracting due to micro-leaks. In some examples, the charging procedure may be performed at the point-of-manufacture, while in other examples, the suction apparatus may be provided in an uncharged state and fully charged at the point-of-use.

In some embodiments, the seal mount 210 may further comprise stabilizers 211 which prevent or resist excessive angular displacement of the slidable seal 207 with respect to the primary axis of the suction apparatus 101. The stabilizers 211 may comprise longitudinal extensions or projections from the seal mount 210. The stabilizers 211 may or may not have an orientation that is generally parallel to the longitudinal movement axis of the seal 207. Also, a stabilizer 211 may be configured to be in sliding contact with the wall of the suction chamber 202 along its entire length, or may be configured to only partially contact the wall of the suction chamber 202. For example, a portion of the stabilizer may curve or angle away from wall of the suction chamber. In some embodiments, the interior of the suction apparatus 101 further comprises a friction-reducing lubricant or a lubricous coating. In other examples, the seal and/or seal mount may have a variable thickness along its perimeter or contact with the wall of the suction chamber. In some instances, an increased thickness may increase the stability of the seal along a dimension of the seal. In some examples, the portion of the seal and/or seal mount with the increased thickness may vary depending upon the transverse dimension intersecting a) the portion of the perimeter and b) the central movement axis of the seal and/or seal mount. Other examples of seals and/or seal mounts with a variable thickness are provided in greater detail below.

Although the reduced pressure therapy device depicted in Figs. 1 to 4 comprises a suction apparatus 101 with separate "activation" and "release" actuators, in other embodiments, a single actuator with an "activation" and a "release" position may be provided. In still other embodiments, no actuators may be provided. In some of the latter embodiments, the suction apparatus may begin to generate reduced pressure once the force from the reset tool is no longer applied. In other examples, the suction apparatus may be configured with activation and/or release mechanisms that may open or close a valve from the coupling or decoupling of the extension tube. For example, the suction apparatus may comprise a slit valve which opens when the extension tube or a connector is inserted into it.

In some embodiments, the suction apparatus may comprise a separate or separatable collection chamber which may be coupled or contained within a housing. The housing may be configured to interface with the collection chamber and self-generate a reduced pressure level within the collection chamber. In some embodiments, the housing further comprises at least one force member that is configured to couple to the seal or seal mount located in the collection chamber. In some embodiments, a reset tool may be used to facilitate the coupling of the collection chamber and the housing and/or to charge the seal. In some embodiments, the collection chamber of the suction apparatus may be separated from the housing, disposed and a new collection chamber may be coupled to the housing. In other embodiments, the collection chamber may be separated from the housing, emptied and/or cleaned, and then re-coupled with the housing. During long-term use of the reduced pressure therapy device, the housing may also be replaced due to wear and tear of the housing or the force member(s).

In some embodiments, a method of applying reduced pressure therapy to an area of damaged tissue is provided, comprising: affixing a sealant layer around an area of tissue to be treated; creating a sealed enclosure around the area of the tissue with the sealant layer; charging or resetting a suction apparatus by positioning a reciprocating member contained in the suction apparatus to an extended position where the effective collecting volume of the suction apparatus is about zero; creating a fluid communication between the sealed enclosure and the suction apparatus; and activating the suction apparatus by drawing back the reciprocating member to a retracted position thereby forcefully expanding the volume of the air originally located within the sealed wound enclosure and generating a reduced pressure level within the sealed enclosure.

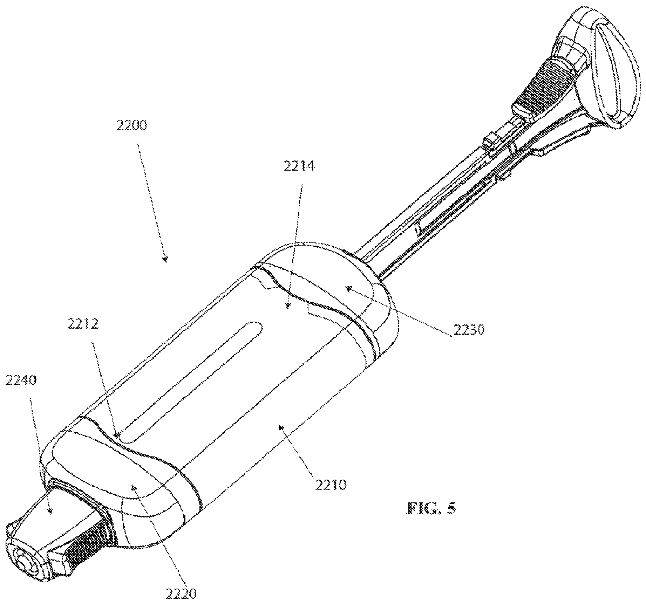

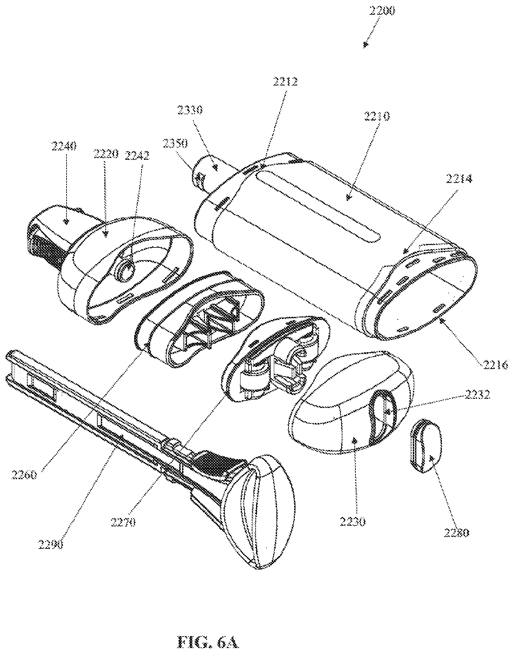

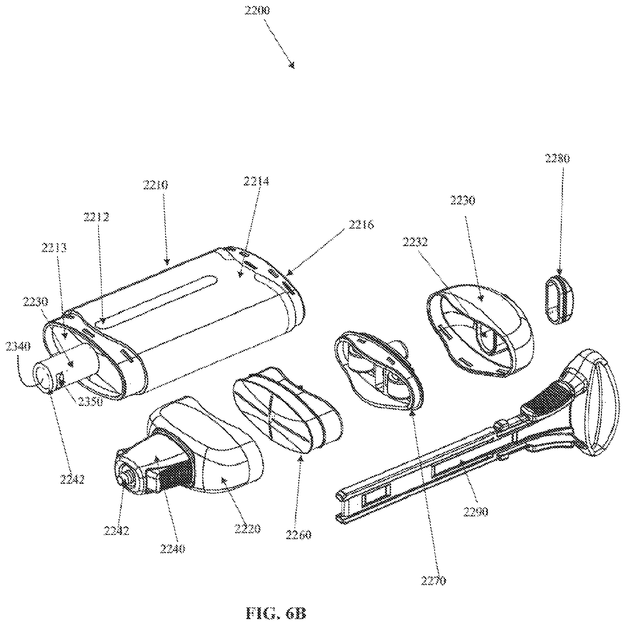

Another embodiment of a suction apparatus 2200 is illustrated in Figs. 5, 6A and 6B. Suction apparatus 2200 comprises a suction chamber 2210 having a distal end 2212 and a proximal end 2214, a front cap 2220 and a rear cap 2230. The front cap 2220 and the rear cap 2230 may be configured to be detachably secured to the distal end 2212 and the proximal end 2214 of the suction chamber 2210, respectively. The proximal end 2212 and/or the distal end 2214 of the suction chamber 2210 may also comprise notches 2360 and 2370, respectively, which may be configured to facilitate coupling to the rear cap 2230 and/or front cap 2220 of the device 2200, respectively. Notches 2372 or apertures may also be provided for attaching the spring assembly 2270 to the suction chamber 2210. A fitting housing 2240 may be coupled to the front cap 2220, enclosing a fitting 2242 that may be configured to connect the suction chamber 2210 with another component of the therapy system (e.g., an extension tube or an attachment port on a sealant layer). The suction chamber may be fabricated from a rigid polymer adapted to maintain the external shape of the suction chamber shape under reduced pressure. In some embodiments, the entire body of the suction chamber may be transparent, thereby permitting visual inspection the quantity and quality of wound exudates contained therein. In other embodiments, the suction chamber may comprise a non-transparent body but with an inspection window.

As mentioned above, the fitting housing 2240 may be configured to removably detach from to the front cap 2220, while in other examples, the fitting housing may be integrally formed with the front cap 2220 or otherwise configured not to be detached once joined. A piston assembly may be movably located within the suction chamber 2210. The piston assembly 2260 may be coupled to a spring assembly secured to the rear cap 2230 of the suction apparatus 2200. In other embodiments, the spring assembly 2270 may also be secured about the proximal opening 2216 of the suction chamber 2210. An opening 2232 may be provided in the rear cap 2230 to permit insertion of a reset tool 2290 which is configured to charge the suction apparatus 2200. Once the suction apparatus 2200 is charged and activated, the reset tool 2290 may be removed, and the opening 2232 on the rear cap 2230 may be closed by a rear cap seal 2280. The rear cap seal 2280 may be any type of seal that may prevent entry of undesired contaminants or other environmental agents (e.g. water during showering) into the suction chamber 2210. In other examples, the rear cap seal may be attached to the rear cap by a tether. In still other examples, the rear cap seal may be configured with a passageway or slit and comprises a deformable material that permits insertion and/or removal of the reset tool and reseals upon removal of the reset tool. In the latter embodiments, the rear cap seal need not be removed before charging or inserted back into the opening after removal of the reset tool.

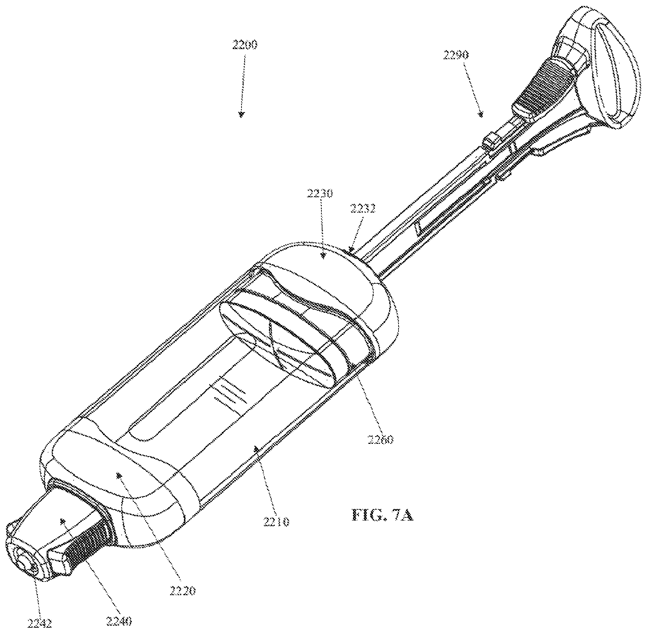

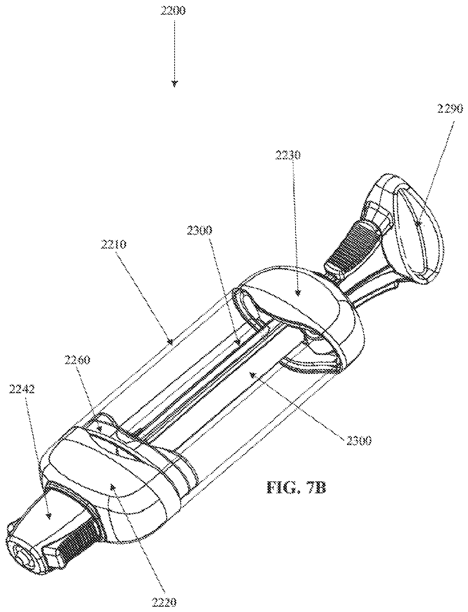

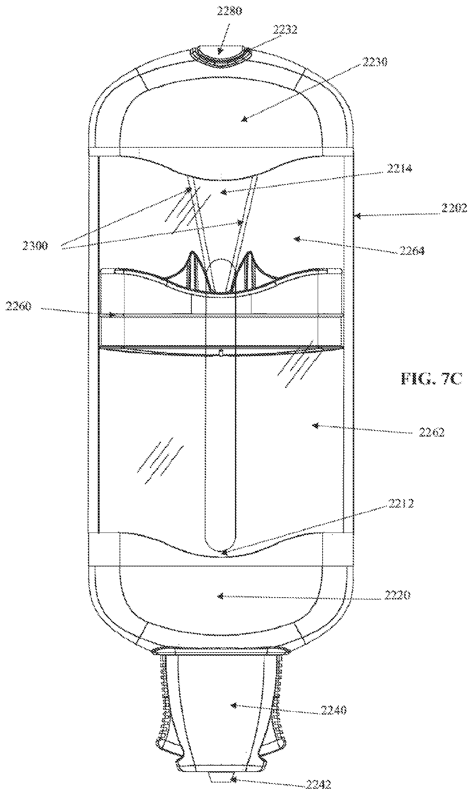

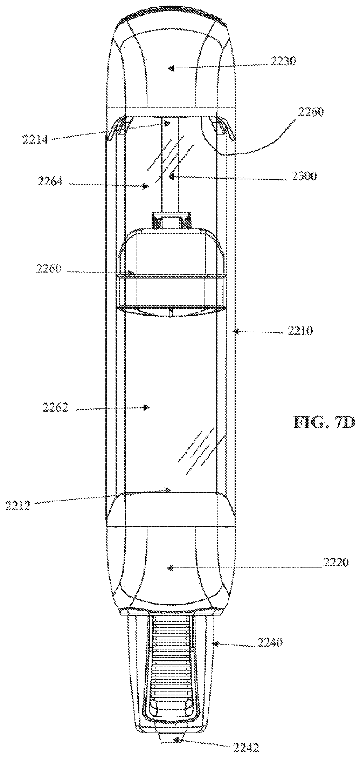

Fig. 7A is a perspective view of the embodiment of the suction apparatus 2200 in a depleted or uncharged configuration and comprising a suction chamber 2210 made of a translucent or optically clear material, with the piston assembly 2260 in a proximal position and the reset tool 2290 inserted into the opening 2232 of the rear cap 2230 but not yet displacing the piston assembly 2260. To charge the suction apparatus 2200, the reset tool 2290 may by further inserted through the opening 2232 of the rear cap 2230 to push the piston assembly 2260 into the suction chamber 2210. Depending upon the particular configuration, the reset tool may be pushed until the piston assembly contacts the distal end wall until it is adjacent the distal end wall of the suction chamber, until the springs are maximally extended, and/or mechanical interference between the reset tool and the rear cap resist further insertion. Fig. 7B depicts the suction apparatus 2200 in the fully charged configuration. The reset tool 2290 has pushed the piston assembly 2260 into a distal position and has extended the springs 2300 coupling the piston assembly 2260 to the spring assembly 2270 and generated potential energy within the springs 2300. Upon removal of the reset tool 2290, the springs 2300 are able to exert a proximal directed force onto the piston assembly 2260, which is capable of generating reduced pressure in the suction chamber 2210 and transmitting the reduced pressure to a sealed wound enclosure coupled to the device 2200. As the suction apparatus applies reduced pressure to the wound enclosure, the piston assembly 2260 moves from its position in the fully charged state (Fig. 7B) across the suction chamber 2210 proximally to its position in the fully depleted state (Fig. 7A). In this process, the suction apparatus is in varying states of depletion (e.g., partially depleted). Figs. 7C and 7D are superior and side elevational views of the device from Fig. 7A in an activated state and with the springs 2300 having partially expended the potential energy from the fully charged configuration. As can be seen when the piston assembly 2260 is in a partially expended position, the suction chamber 2210 may be subdivided by the piston assembly 2260 into a collection chamber 2262 and a working chamber 2264, where the collection chamber 2262 is the space between the piston assembly 2260 and the distal end wall 2213 of the suction chamber 2210, and the working chamber 2264 is the space between the proximal end 2214 of the suction chamber 2210 and the piston assembly 2260 which contain the springs 2300. When the suction apparatus is in the charged (e.g., fully charged or at least partially charged) configuration, the volume of the collection chamber may be about zero, or sometimes less than about 5 cc. In some instances, upon activation of the charged device, the collection chamber may increase in volume up to about 3%, sometimes about 5% and other times about 10% or even about 20% until the force exerted by the springs 2300 is counterbalanced by the force generated by the reduced pressure in the collection chamber 2262.

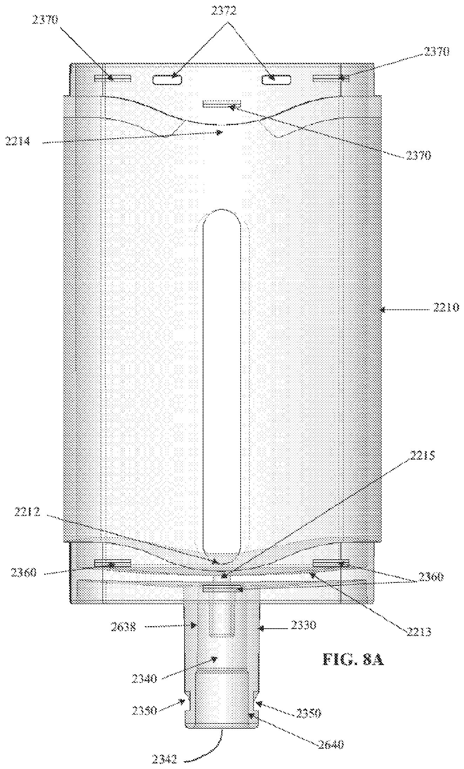

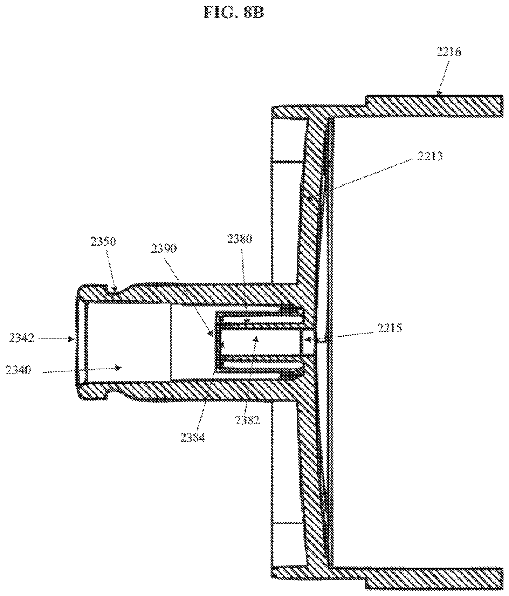

Fig. 8A provides a detailed superior view of the suction chamber 2210 and Fig. 8B provides a cross-sectional view of the distal portion of the suction chamber 2210 from Fig. 8A. As may be seen in the perspective views in Figs. 5 to 7B, the suction chamber 2210, may comprise a non-circular cross-sectional shape with respect to a transverse plane to the movement axis of the piston assembly, which in some configurations lies between the distal end 2212 and proximal end 2214 of the suction chamber 2210. In other examples, the cross-sectional shape of the suction chamber may have any of a variety of other types of geometric configurations (e.g., cylindrical, rectangular, etc.). As mentioned previously, the distal end wall 2213 of the suction chamber 2210 may further comprise a distal opening to permit communication with the suction chamber. The distal end wall 2213 of the suction chamber 2210 may further comprise a conduit 2330 or other extension structure. The conduit 2330 comprises a conduit lumen 2340 with a conduit opening 2342 which are in fluid communication with the collection chamber 2262 of the suction chamber via the distal opening 2215 of the distal end wall 2213. The conduit 2330 may comprise any of a variety of notches 2350, grooves or flanges, which may facilitate attachment of the conduit 2330 to one or more components associated with the fitting housing 2240.

Although a user-controlled valve may be provided in some embodiments to open or close fluid communication with the suction chamber, in some examples, the fluid communication may be controlled automatically by the coupling and/or decoupling of the device components. For example, the conduit 2330 of the device 2200 may also comprise an inner conduit 2380 located in the main conduit lumen 2340, the inner conduit 2380 comprising an inner conduit lumen 2382 and an inner conduit opening 2384. Referring to Fig. 8B, a chamber slit seal 2390 may be located about the inner conduit opening 2384. In its base configuration, the chamber slit seal 2390 may be configured with a normally closed configuration to block fluid communication through the conduit 2330. In some examples, a chamber slit seal 2390 may be opened by inserting a structure through the seal to deform it and maintain the patency of the opening formed in the seal. As will be explained in greater detail below, in other examples, such as the slit seal 2390 in Fig. 8B, the slit seal 2390 may be configured to be pushed over, around, and/or down toward the base of the inner conduit 2380 when a complementary structure is inserted into the main conduit lumen 2340.

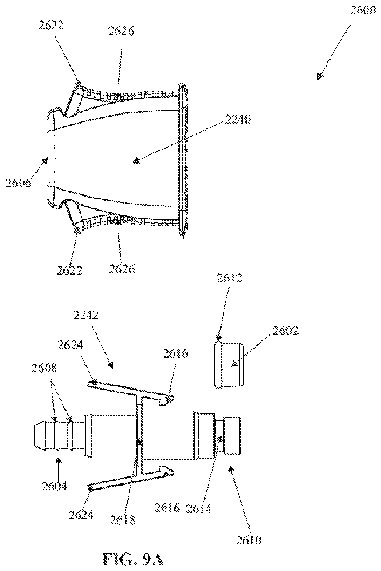

Fig. 9A is a top component view of a fitting assembly 2600, comprising the fitting housing 2240, a fitting 2242 and a fitting slit seal 2602. As mentioned previously, the fitting housing 2240 may be configured to permanently or detachably couple to the front cap 2220 of the device 2200, or may be integrally formed with the front cap. In the embodiment shown in Fig. 9A, fitting 2610 comprises a connector section 2604 that is accessible through an opening 2606 in the fitting housing 2240 and permits a complementary fit with the connector of another component. For example, connector section 2604 may be coupled to a connector of an extension tube or the attachment port of a sealing layer with a snap fit or an interference fit. In the specific example in Fig. 9A, the connector section 2604 comprises multiple flanges 2608 which may be used to provide a resistance fit with tubing, but may also be used with a complementary connector to form a complementary interfit.

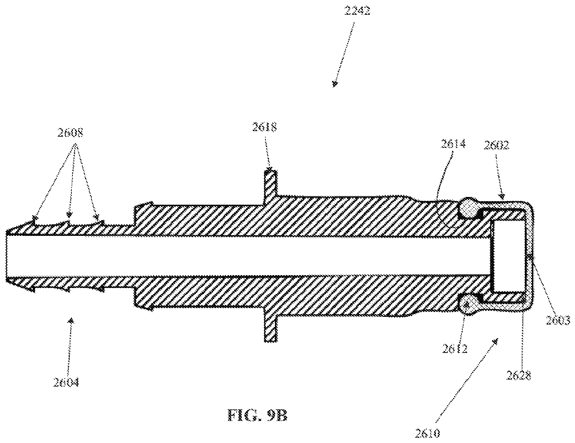

Referring to Figs. 26A and 26B, the fitting 2242 may also comprise a chamber connector 2610 with a fitting slit seal 2602. When the device is assembles, the chamber connector 2610 may be located within the front cap 2220 of the device 2200, but the particular location may vary with the particular embodiment. The fitting slit seal 2602 may comprise a distal ring 2612 with an inner profile configured to engage a groove 2614 on the chamber connector 2610 of the fitting 2242. The outer profile of the seal 2602 and/or the distal ring 2612 may be configured to seal against the inner surface main conduit lumen 2340. The fitting slit seal 2602 may also comprise a slit that provides a deformable passageway through the seal 2602. Thus, in some embodiments, the fitting slit seal 2602 may be configured to both form an airtight seal between the chamber connector 2610 and the conduit lumen 2340 of the suction chamber 2210 and also to control fluid communication through the fitting assembly 2600. Fig. 9B illustrates a side cross sectional view of fitting 2610 coupled to the fitting slit seal 2612 at the fitting's proximal end.

Referring back to Fig. 9A, fitting assembly 2600 may also comprise an interlocking structure that comprises at least one resilient tab 2616 that is disposed on and project outwardly from a base member 2618 coupled or integrally formed with the fitting 2242. When the fitting assembly 2600 is coupled to the suction chamber 2210, the tabs 2616 are configured to engage complementary recesses (2350 in Figs. 8A and 8B) on the conduit 2330 of the suction chamber 2210. An interlocking mechanism may resist or prevents inadvertent decoupling of the fitting 2242 from the suction chamber 2210. The fitting housing 2240 may further comprise one or more release structures or buttons 2622 that are coupled to or interface with the levers 2624 of the projecting tabs 2618. Depressing the buttons 2622 will release the interlocking mechanism by displacing the tabs 2616 from the notches 2350 on the suction chamber 2210 and permit decoupling of the fitting 2242 and fitting housing 2240 from the front cap 2220 and the suction chamber conduit 2330. The release buttons 2622 may comprise one or more textured gripping surfaces 2626 that may facilitate manual connection or disconnection of the fitting 2242.

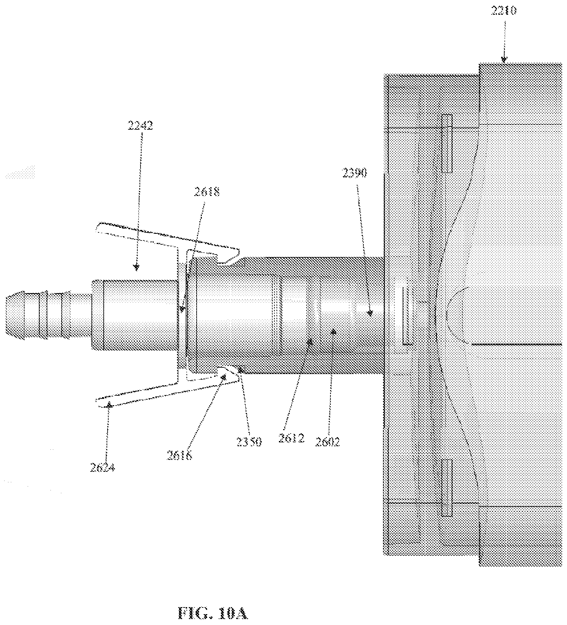

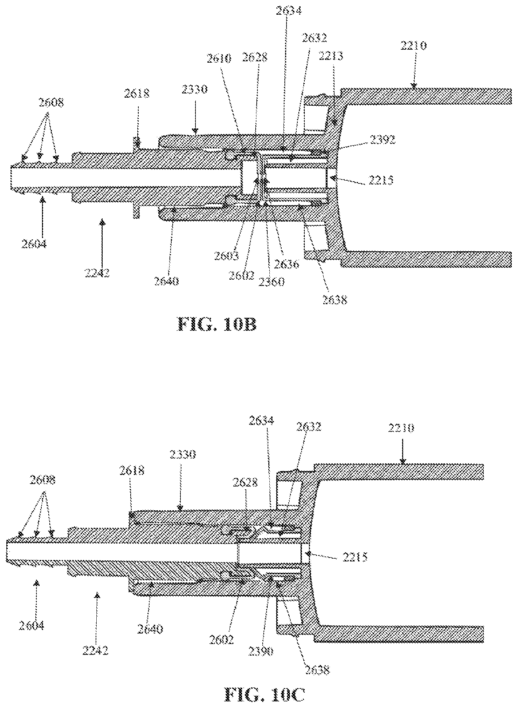

Fig. 10A is a schematic superior cut-away view of the suction chamber 2210 and the fitting 2242 of the fitting assembly 2600 when the fitting 2242 is fully inserted into the conduit 2330. As illustrated, the tabs 2616 projecting from the base member 2618 of the fitting 2242 form an interfit with the notches 2350 on the surface of the suction chamber conduit 2330. Figs. 10B and 10C are side cross sectional views of a portion of the suction chamber 2210 and the fitting 2242, before and after the fitting 2242 has been fully seated into the conduit 2330. Figs. 10B and 10C further illustrate the connecting mechanism between chamber slit seal 2390 on the inner conduit 2380 and fitting slit seal 2602 of the fitting 2242. In Fig. 10B, when fitting 2242 is inserted into the conduit 2330, the fitting slit seal 2602 initially contacts chamber slit seal 2390, which is mounted on a seal base 2392. As illustrated in Fig. 10C, further insertion causes the edge 2628 of the chamber connector 2610 to exert a force along the perimeter 2660 of the chamber slit seal 2390. An inner gap 2632 and/or an outer gap 2634 about the chamber slit seal 2390 provide space for the chamber slit seal 2390 to deform or compress away from the edge 2628 of the chamber connector 2610. This results in the enlargement of the opening or slit 2636 of the chamber slit seal 2390 as it is pushed proximally away from the inner conduit opening 2384. In some examples, the inner and outer gaps 2632 2634 may also reduce the frictional resistance of the chamber slit seal 2390 against the inner conduit 2380 or the surface of the conduit lumen 2340, respectively. As the fitting 2242 is further inserted into the conduit lumen 2340, the exposed inner conduit 2380 penetrates through the slit 2603 of the fitting slit seal 2602, thereby opening fluid communication from the suction chamber 2210, through the distal opening 2215 of the suction chamber 2210, through the inner conduit 2380 and through the fitting 2242. In the embodiment depicted in Figs. 10A to 10C, the tabs 2616 and the notches 2350 of the locking mechanism may be used to provide rotational alignment of the between the fitting slit seal 2602 and the chamber slit seal 2390, if needed. This may be useful where the slits of the seals 2602 and 2390 are single linear slits. In other configurations where the slits are multiple radial slits, rotational alignment may or may not affect the patency of the fluid communication.

When fitting 2242 is decoupled from the suction chamber conduit 2330, of the withdrawal of the inner conduit 2380 from the fitting slit seal 2602 results in closure of the fluid passageways to the sealed wound and may limit air entry into the wound during decoupling. As the fitting 2242 is further separated, the edge 2628 of the chamber connector 2610 is withdrawn and the chamber slit seal 2380 is able to elastically revert back to a closed position to seal the suction chamber 2210. In some embodiments, chamber slit seal 2380 is able to elastically revert back to a closed position with the aid of a coaxially mounted coil spring. Although both seals 2602 and 2390 are closed, the outer surface of the fitting slit seal 2602 continues to form a seal with the conduit lumen 2340 until further separation occurs. As may be seen in Figs. 10B and 10C, the conduit lumen 2340 of suction chamber 2210 has a non-uniform diameter along it longitudinal length, and may comprise a proximal segment 2638 having a reduced diameter relative to the distal segment 2640. The transition in diameter between the proximal and distal segments 2638 and 2640 may be gradual or stepped. The conduit lumen 2340, for example, comprises at least one step transition region 2642 between the segments 2638 and 2640. In some examples, step transition region may provide different tactile feedback compared to gradual transitions.

The slit seal may be fluid impervious and may be fabricated from any of suitable resilient materials, such as, but not limited to, synthetic elastomer, silicone rubber, or natural rubber. The seal material may be compatible with wound exudates that may be collected by the suction chamber during a reduced pressure treatment. The seal material may be sterilized by treatment of radiation, steam, ethylene oxide or other suitable techniques known to those skilled in the art.

Alternatively or additionally, the distal conduit of a suction chamber and/or the detachable fitting or distal connector may comprise a one-way check valve within the housing of the connector. For example, a one-way check valve may be integrated within the detachable connector, which is configured to allow fluid to flow into the suction chamber (but not out of the chamber). FIGS. 14A and 14B depicts one embodiment comprising a connector 3200 with a housing 3201 and a one-way vacuum check valve 3202 within the housing 3201. The one-way check valve 3202 may be located between a proximal opening 3206 and a distal opening 3204 of the connector, where the proximal opening 3206 is configured to detachably connect to the distal end of a suction chamber. The one-way check valve 3202 is oriented such that fluid (gas, liquid and/or suspended materials) to flow into, but not out of, the suction chamber. The permitted fluid flow across the one-way check valve 3202 is represented by the arrow 3203 in FIG. 14B. In some variations, the one-way check valve 3202 may be a duckbill valve, but other one-way valves mechanisms may also be used including ball check valves, diaphragm check valves, tilting disc check valves, and the like. The one-way check valve 3202 may be selected with an opening pressure in the range of about 1 mm Hg, to about 5 mm Hg, and sometimes about 5 mm Hg, to about 15 mm Hg, or about 15 mm Hg, to about 30 mm Hg, and may sometimes be about 10 mmHg, or less than 1 mmHg, or about 20 mmHg .

In variations of suction devices that comprise a connector with an integrated one-way vacuum valve, it may be difficult to recharge or reset the suction chamber after it has been exhausted when the system does not include a venting mechanism once all parts are connected. It may also be difficult to reset the suction chamber when the device is initially attached to a dressing and has removed the air present below the dressing. With the initial application of a contact layer and dressing over a wound, an amount of air is often present below the dressing or in the dressing itself (e.g., when the dressing has a foam component which is initially uncompressed). After initially attaching and activating the suction device, a significant amount of the air "deadspace" may be removed from underneath the dressing and pulled into the cartridge as the contact layer compresses during initiation of negative pressure. This action may limit the remaining available volume for exudate collection and/or affect the duration of negative pressure delivery of the cartridge. In some instances, the user may separate the detachable fitting from the suction chamber so that the suction device may be reset by re-inserting the reset tool and pressing the slidable seal distally (i.e., forward) to expel the air collected from underneath the dressing. After the suction device has been recharged, the reset tool may be removed to re-initiate negative pressure delivery. Recharging the suction device may be difficult particularly if during the removal of the air from underneath the dressing, wound exudates are taken up into the suction chamber and/or if an absorbent member within the suction chamber blocks the distal port (i.e., vacuum conduit) of the suction chamber. In such cases, it may be undesirable to attempt to expel the air collected from underneath the dressing, since it may expose the user to collected exudates in an uncontrolled manner if also expelled during the recharging process.

In some variations, a suction device may further comprise an additional or different one-way check valve in fluid communication with the collection chamber, where this one-way valve may be configured to allow fluid (e.g., air) to flow out of, but not into, the collection chamber. For example, this one-way valve may allow air to flow out of the collection chamber into the working chamber and/or outside of the device. This one-way valve may help to alleviate the challenges described above by allowing the air collected from underneath the dressing to be expelled without removing the connector. This one-way valve may be configured such that when there is negative pressure within the suction chamber, the one-way valve is closed (e.g., when the piston assembly is moving in a proximal direction and maintaining negative pressure within the suction chamber), but when positive pressure is applied to the chamber (e.g., when the piston assembly is moving in a distal direction), the one-way valve may open to allow fluids (e.g., air) to flow out of the suction chamber. For example, this one-way valve may be any suitable check valve, such as an umbrella valve, flap valve, cross slit valve, dome valve, ball valve, duckbill valve, or blow-by seal. In some variations, the one-way valve in the distal conduit of the suction chamber and/or the detachable fitting or distal connector may be the same type (e.g., have the same check valve mechanism) as this other one-way valve, while in other variations, the one-way valve associated with the distal conduit may be a different type (e.g., have a different check valve mechanism) from the other one-way valve. For example, the one-way valve (in the distal conduit and/or the detachable connector) may be a flap valve, while the other one-way valve may be a duckbill valve. In other examples, both of the one-way valves may be duckbill valves. The other one-way check valve may have an opening pressure that is the same or different as the opening pressure ofthe one-way check valve associated with the distal conduit. The other one-way valve may have a lower opening pressure than the one-way check valve associated with the distal conduit. The second one-way valve may be selected with an opening pressure in the range of about 1 mm Hg to about 5 mm Hg, and sometimes about 5 mm Hg to about 15 mm Hg, or about 15 mm Hg to about 30 mm Hg, and may sometimes be about 10 mmHg, or less than 1 mmHg, or less than 5 mmHg (e.g., about 2 mmHg or 3 mmHg), or about 20 mmHg or about 50 mmHg or about 100 mmHg. Valves with different opening or crack pressures may be selected at least partially based on their location on the suction device. For example, a one-way valve located along a side wall of the suction chamber may have opening or crack pressures in the range of about 1 mmHg to about 150 mmHg or more, e.g., from about 2 mmHg to about 5 mmHg, from about 5 mmHg to about 100 mmHg, or about 2 mmHg, 3 mmHg, about 20 mmHg to about 125 mmHg, or about 50 mmHg to about 100 mmHg. The opening or crack pressure of a valve may also vary depending on the type and quantity of lubricant applied to the valve. A valve lubricant may help reduce the leakage through the valve, and in the case of a silicone valve, it may also help prevent the silicone from binding to each other. In some variations, the valve lubricant may affect (e.g., interfere) the air flow through the valve. For example, an unlubricated valve may have an opening or crack pressure of about 3 mmHg, and a lubricated valve of the same type may have an opening pressure of about 15-45 mmHg (depending at least in part on the quantity and type of lubricant, as well as the time between which the valve has previously been opened). The opening or crack pressure of a valve may also vary during the life of the valve. For example, as a valve sits unused over a period of time, its opening pressure may increase. In some variations, the one-way valve may only be open when a reset tool is inserted in the suction device and advanced in a distal direction to displace the piston assembly in the suction chamber. For example, the one-way valve may be a button valve that may be activated only when the reset tool is inserted into the suction device. The opening force of a button valve may be about 2 lbf or less. Optionally, an opening of the one-way valve (i.e., the opening that faces the interior of the collection chamber) may be covered with a hydrophobic filter and/or a mesh to prevent exudates from exiting, and may optionally be covered with a charcoal filter.

The other one-way valve may be located along a lateral side wall 3210, distal side wall 3212 and/or shoulder of the suction chamber, and/or along the slidable seal of the piston assembly, as schematically depicted in FIG. 14C. For example, this one-way valve may be located at a distal portion of the lateral side wall 3210 such that the valve does not interfere with the ability of piston assembly to generate negative pressure (e.g., the valve does not obstruct the path of the piston assembly). FIG. 14D depicts an example of a suction device 3211 that may comprise a one-way valve 3214 at a distal wall 3216 of the suction chamber 3213, where the valve 3214 may be configured to allow fluids to flow out of, but not into, the suction chamber. The permitted fluid flow across the one-way valve 3214 is represented by the arrow 3215 in FIG. 14D.

Alternatively or additionally, a one-way valve may be located in the slidable seal of a piston assembly, which may allow air to exit the distal portion of the suction chamber (e.g., the collection chamber) when the reset tool is advanced distally (i.e., forward), but does not permit air to enter the suction chamber when the suction chamber is under negative pressure. In some variations, the one-way valve may be integrally formed (e.g., molded) with the slidable seal, while in other variations, the one-way valve may be separately formed and subsequently assembled with the slidable seal. Optionally, a lubricant (e.g., a silicone lubricant) may be applied on the one-way valve to help prevent a slit seal in the one-way valve from self-sealing or sticking. In some variations, a filter (e.g., a hydrophobic filter) may be located over the one-way valve, which may provide allow air to pass through the one-way valve, but may limit the ability of liquids and/or solids to pass through the one-way valve and exit the collection chamber. Alternatively or additionally, other types of barriers may be used to limit liquid and/or solids from flowing across the one-way valve, while allowing air to pass through. For example, a cover flap (e.g., made of a gas and liquid impermeable elastic material or a porous foam) may be located over the valve (and/or located over the optional filter), which may help to prevent liquids and/or solids from contacting the valve (and/or filter) while allowing air to pass through. Alternatively or additionally, a foam layer and/or impermeable membrane layer may be disposed between the slidable seal and the contents of the collection chamber. Some variations of slidable seals may comprise two or more one-way valves, each of which may have a filter and/or flap located over it. While slidable seals with one or more one-way valves are described as being used with suction devices having a distal connector comprising a one-way valve, it should be understood that slidable seals having one or more one-way valves may be used in any of the suction devices described herein, including suction devices that do not have a distal connector comprising a one-way valve.

FIGS. 14E and 14F depict a perspective and side view of one variation of a slidable seal 3224 that has a one-way valve 3220 located across it. The one-way valve 3220 may be configured to allow fluids (e.g., air) to flow from the distal portion of the suction chamber (e.g., the collection chamber) to a proximal portion of the suction chamber (e.g., the working chamber), but not from the proximal portion to the distal portion of the suction chamber. The permitted fluid flow across the one-way valve 3220 is represented by the arrow 3221 in FIG. 14F. FIGS. 14G to 14I depict a one-way valve 3230 as claimed, located in the slidable seal 3231 of a piston assembly 3234. FIG. 14G depicts the proximal side of the slidable seal 3231 (i.e., the side of the slidable seal that faces the working or non-vacuum chamber. The one-way valve 3220 is configured to allow fluids (e.g., air) to flow from the distal portion of the suction chamber (e.g., the collection chamber) to a proximal portion of the suction chamber (e.g., the working chamber), but not from the proximal portion to the distal portion of the suction chamber. The permitted fluid flow across the one-way valve 3230 is represented by the arrow 3232 in FIG. 14H. The valve 3230 is configured to be opened only when a reset tool 3236 is inserted into the suction device and pressed against it. In some variations, the one-way valve 3230 may have a protrusion 3238 that may engage with the reset tool 3314, and such that when the reset tool is engaged with the valve protrusion and pushed distally, the one-way valve is opened (FIG. 14I). Although only a single one-way valve for the collection chamber is depicted in FIGS. 14C to 14F, in other variations, two or more one-way valves for the collection chamber may be provided.

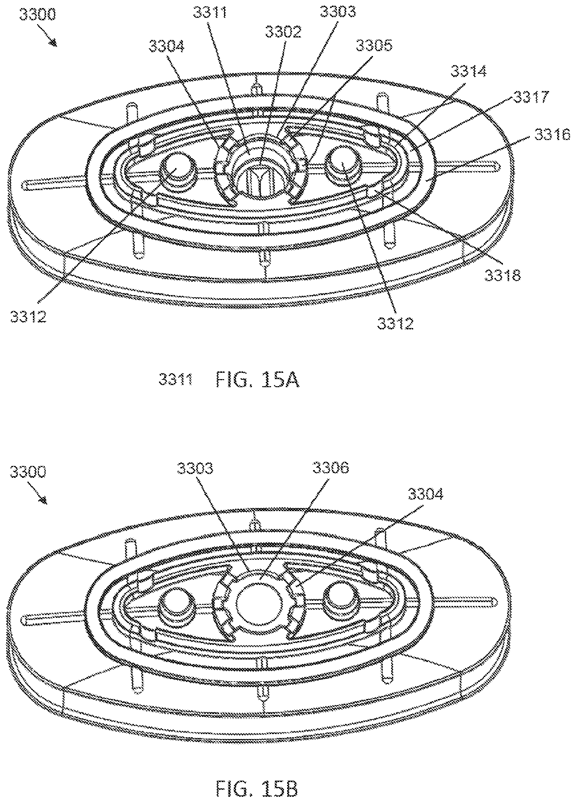

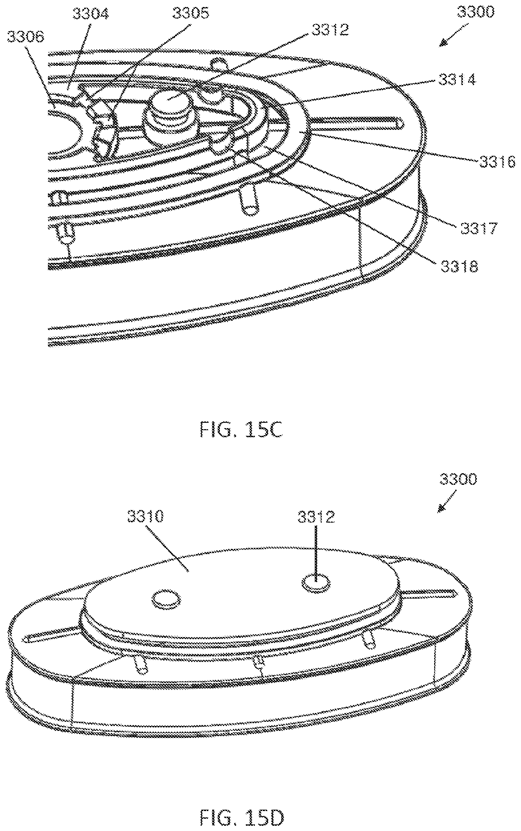

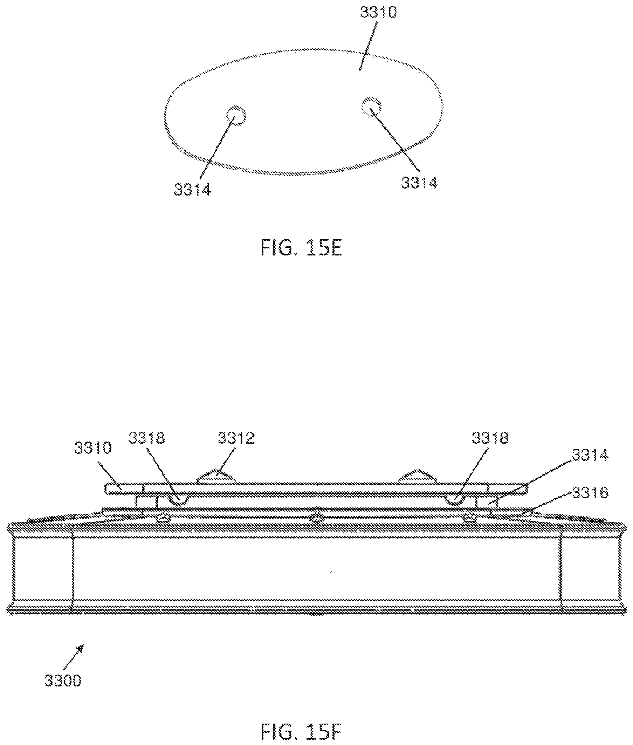

In some variations, the distal side of the slidable seal (i.e., the side that faces the collection or vacuum chamber) may comprise a filter configured to allow air to pass through, but prevents or greatly limits the ability of liquids and/or solids to pass. This may help to clean the air that is expelled proximally through the one-way valve. FIGS. 15A and 15B depict the distal side (e.g., the side that faces the collection chamber or vacuum chamber) of one variation of a slidable seal 3300 with a one-way valve 3302 (e.g., a duckbill valve) integrally formed with the slidable seal. There may be an opening 3303on the slidable seal that is connected to a conduit 3311 that passes through the slidable seal and connects with the second one-way valve 3302. The slidable seal may comprise a semi-circular or circular ringshaped retaining structure or protrusions 3304 around the opening of the second one-way valve 3302 that may be sized and shaped to retain a filter 3306 therebetween. The retaining structure 3304 may comprise one or more protrusions that are spaced apart by openings or gaps 3305 (e.g., at least 1, 2, 3, 4, 5, 6, 8, 10, 12, 14, 16, 20, or 24 gaps, etc.). The filter may be retained over the one-way valve by mechanical engagement with the protrusions of the retaining structure 3304 (e.g., friction-fit, snap-fit, screw-fit), it may alternatively or additionally be retained over the one-way valve by adhesives, melding, and/or any other suitable mechanisms that provide a liquid-tight seal with the slidable seal 3300 over the one-way valve 3302. FIG. 15A depicts the slidable seal 3300 without the filter 3306, while FIG. 15B depicts the filter 3306 retained by the semi-circular protrusions of the retaining structure 3304. While the filter 3306 and the corresponding retaining structure 3304 are depicted as generally circular, it should be understood that they may be any suitable shape or size, e.g., rectangular, triangular, oval- or ellipse-shaped, etc. The filter may be a hydrophobic filter or a gas permeable but liquid impermeable filter or a gas and liquid permeable filter. For example, the filter may be made of a compressed foam (such as "3-1000" compressed foam, felted foam with a 100 + 20 ppi pre-felting porosity). In some variations, the filter may comprise pore sizes that are smaller than the shortest bacterial cross-sectional dimension. For example, the pores may have a diameter of about 0.1 micron to about 7 microns, e.g., about 0.3 micron, about 0.5 micron, about 1 micron, about 3 microns, about 5 microns, etc. Pores may also be sized and/or treated to capture viruses and/or fungi spores.

Any of the suction devices described herein having a one-way valve in fluid communication with the collection chamber for expelling air may optionally comprise a bendable valve cover or flap (e.g., a shut-off flap) may be disposed over the filter which may help to act as a barrier between the filter and the exudates (and/or absorbent materials retaining wound exudates) in the collection chamber. This may help prevent the filter from being clogged with liquids and/or solids, which may impede the ability of air to pass through the filter. In some variations, the flap may be made of a non-absorbent material. For example, the flap may be made of an air and liquid impermeable material such as silicone rubber, thermoplastic elastomer, polyisoprene rubber, natural rubber, and/or any elastomeric material. In some variations, the flap may be attached to the slidable seal. The flap may have a first configuration where air is permitted to pass from the suction chamber through the filter and the valve and a second configuration where air is not permitted to pass from the suction chamber through the filter and the valve. In both configurations, the flap forms a non-absorbent barrier between the exudates and/or super absorbent materials within the suction chamber and the filter and valve of the slidable seal. In the first configuration, the flap may be in a position where air gaps/openings in the slidable seal are not obscured while in the second configuration, the flap may be in a different position that obscured the air gaps/openings in the slidable seal.

One example of a flap that may be coupled to a slidable seal to perform the functions described above is depicted in FIGS. 15D-15E. The flap 3310 may be made of a flexible material, which may be gas and liquid impermeable, such as silicone (e.g., 50 Shore A silicone), urethane, rubber, elastomer, thermoplastic elastomer, or any such flexible, liquidproof material. The surface of the flap 3310 may be smooth or textured. The slidable seal 3300 may comprise one or more protrusions or barbs 3312 that may fit within corresponding openings 3314 in the flap 3310 to retain the flap over the filter. Alternatively or additionally, the flap 3310 may be secured to the slidable seal by adhesives, staples, fasteners, etc. In some variations, the flap may be integrally molded with the slidable seal. The slidable seal 3300 may also comprise an inner wall 3314 circumscribing the valve opening and optionally the barbs and an outer wall 3316 circumscribing the inner wall. The inner wall 3314 may comprise one or more openings or gaps 3318 around its perimeter (e.g., 1, 2, 3, 4, 5, 6, 8, 10, 12, 14, 16, 20, or 24 gaps, etc.). The height of the inner wall 3314 may be greater than the height of the outer wall 3316. For example, the height of the inner wall 3314 may be at least 0.2 inches greater than the height of the outer wall 3316. The space between the inner wall 3314 and the outer wall 3316 may form a channel 3317 that may act as a buffer between the contents of the collection chamber and the filter. When the flap 3310 is attached to the barbs 3312, in the absence of any additional pressure on the flap, the flap rests on the inner wall 3314,with little or no contact with the outer wall 3316, as depicted in the side view of the slidable seal (FIG. 15F). This permits air to pass from the collection chamber through the openings or gaps 3318 of the inner wall, through the openings or gaps 3305 of the retaining structure 3304, and through the filter to exit the second one-way valve when the slidable seal is advanced forward/distally. The flap may be kept elevated above the outer wall by the increased height of the inner wall relative to the outer wall, and/or the height of the protrusions of the retaining structure 3304. Elevating the flap above the second one-way valve opening may help reduce the incidence of the flap inadvertently shutting off the second one-way valve as the slidable seal is advanced during re-activation. At the same time, the flap may act to provide a barrier between the filter and the contents of the collection chamber to help reduce the likelihood that liquids and/or solids in the collection chamber will contact the filter and/or the second one-way valve. Alternatively the outer wall may be about the same height or taller than the inner wall (e.g., when used with a textured flap).

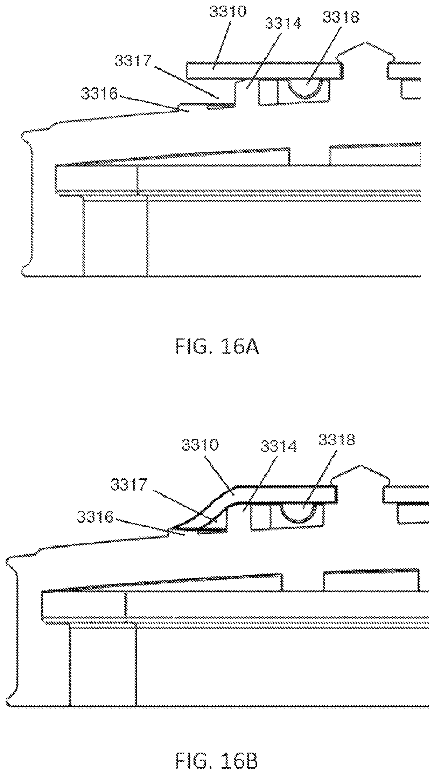

FIGS. 16A-B depict the different configurations of the slidable seal flap during use. FIG. 16A depicts a first configuration where the flap 3310 rests on the inner wall 3314 with little or no contact with the outer wall 3316. The slidable seal flap may be in this first configuration while the slidable seal is being advanced forward/distally within the suction chamber (e.g., during resetting of the suction device), prior to contacting, or only lightly contacting, the contents (e.g., wound exudates and/or super absorbent materials and/or distal wall) of the collection chamber. In this configuration, air may be expelled through the second one-way valve, while reducing the likelihood of the filter contacting the contents of the collection chamber. Such configuration may also allow air collected from underneath a dressing to be expelled at the initiation of negative pressure therapy without removing the detachable connector. FIG. 16B depicts a second configuration where the flap 3310 contacts the outer wall 3316 such that the perimeter of the flap creates a liquid-tight seal with the outer wall. The slidable seal flap may be in this second configuration when the piston assembly is advanced forward/distally within the suction chamber (e.g., during resetting of the suction device) and contacts with sufficient pressure the contents of the collection chamber (e.g., wound exudates and/or absorbent materials and/or distal wall of the suction chamber). For example, during re-activation of the suction device to purge air collected from underneath the dressing, the piston assembly may be advanced distally into an absorbent pad in the collection chamber. Such pressure against the pad may cause the perimeter of the flap to deflect towards the outer wall to form a seal. This may help to reduce the likelihood that any liquids (e.g., wound exudates) that may have been absorbed by the pad and/or the pad itself and/or any activated super absorbent material will contact the filter and/or the one-way valve. In the event that a small quantity of liquids and/or super absorbent material bypasses the perimeter of the flap before it seals with the outer wall, the channel 3317 between the outer wall and inner wall may provide an intermediate buffer or retaining region for the liquids and/or super absorbent material. This may help prevent such liquids and/or materials from contacting the filter. The shut-off mechanism described above is merely one example of a shut-off mechanism for a one-way valve that is attached to a slidable seal is described above. It should be understood that any of the shut-off mechanisms described below may additionally or alternatively be used with a one-way valve located in a slidable seal.

Optionally, in some variations, a suction device may comprise an additional sheet or layer of material may be provided between the flap and the filter associated with the one-way valve. In some variations, the additional sheet or layer of material may be located between the flap and the slidable seal. For example, the additional sheet may be a filter or hydrophobic material, and/or may be a porous foam layer. Including such layer(s) between the flap and filter associated with the one-way valve may help to prevent the flap from adhering undesirably to the over the valve opening. For example, these layers may help prevent the flap from inadvertently adhering to the slidable seal. Such features may be especially useful in embodiments where the flap is mounted generally flush to the surface of the slidable seal, and does not stand off from the surface of the slidable seal by way of an elevated inner wall. However, in some variations, a stand-off may be located on the distal surface of the slidable seal as an additional precaution to keep the flap and the foam from compressing against the one-way valve during re-activation of the suction device. The hydrophobic material, and/or foam layer and/or stand-off may have one or more apertures that is configured to be aligned with the one-way valve opening so that air flow through the valve is not obstructed by these structures. In some variations, the hydrophobic material, and/or foam layer and/or stand-off may be located such that it does not interfere with air flow across the valve. Alternatively or additionally, there may be a plurality of silicone flaps located over the filter and/or one-way valve opening (e.g., attached to the slidable seal), with or without the foam and/or hydrophobic sheet materials.

The flap as depicted and described above is attached to the slidable seal, however, in other embodiments, the flap may not be attached to the slidable seal. For example, the flap may be coupled to a super absorbent material (e.g., an absorbent pad) within the collection chamber. The flap may be coupled to a proximal portion of the super absorbent material using adhesives or tie-downs (e.g., strips made of porous materials or sutures, etc.) such that when the slidable seal is advanced forward/distally (e.g., during re-activation or resetting of the suction device), the slidable seal contacts the flap without contacting the super absorbent material. Alternatively or additionally, an impermeable adhesive film may be adhered to the super absorbent material.

While the flap has been described above as being made of a flexible and liquid-impermeable material attached to the slidable seal, a flap may also be made of other materials and may or may not be attached to the slidable seal. In some variations, there may be no flap at all. For example, the suction device may comprise a porous foam material disposed between the slidable seal and a super absorbent material located within the suction chamber. In some variations, the porous foam materials may be in the form of a sheet or thickened in the form of a block, and/or may be felted foam. The pores in the foam may allow air to pass through to the filter and out of the valve while the thickness of the foam layer may maintain a physical separation between the wound exudates and/or super absorbent material and the filter. The foam may be attached to the slidable seal, or may not be attached to the slidable seal, and may instead reside in the collection chamber, optionally in contact with the chamber walls and/or attached to a super absorbent material within the collection chamber. The foam may have any permeability (e.g., from about 30 ppi to about 100 ppi, about 60 ppi or about 80 ppi, greater than 100 ppi) and/or thickness (e.g., about 1/16 inch to about 1.5 inches, about 1/4 inch, about 1/8 inch). In some variations, a sheet of liquid impermeable material may be provided between the foam and the slidable seal and/or between the foam and the super absorbent material. For example, a sheet of polycarbonate (e.g., about 1/32 inch thick) or a hydrophobic filter sheet may be located between the foam flap and the super absorbent material or between the foam flap and the slidable seal. In some embodiments, the foam layer may be attached to the slidable seal or super absorbent material using an adhesive (e.g., a pressure sensitive adhesive) and/or any suitable mechanical attachment mechanism (e.g., staples, barbs, clasps, cables, tie-downs, etc.). In still other embodiments, instead of a flap or a foam layer, a thick protrusion or bumper may be attached to or integrally molded with the slidable seal so that the bumper maintains a space between the filter in the slidable seal and the exudates and/or super absorbent materials within the collection chamber. Any of the oneway valves described herein, regardless of their number and location on the slidable seal and/or side walls and/or distal walls of the suction chamber, may comprise a filter and/or shut-off flap and/or foam layer as described above.

Any of the suction devices described herein may comprise a fluid retention assembly comprising absorbent materials within the collection chamber so that when the exudates come into contact with the absorbent material, it is absorbed by the material and retained and/or sequestered within the collection chamber. In some variations, the fluid retention assembly may comprise a super absorbent material, such as a super absorbent polymer (e.g., fiber-based polymers or particulate-based polymers) that may or may not contain antimicrobial material. Additional description of super absorbent materials that may be used with a suction device are provided in U.S. Appl. No. 13/175,744, filed July 1, 2011. Optionally, the fluid retention assembly may also comprise a screen or mesh that may be used to sequester the absorbent material in a certain portion of the suction chamber. The screen or mesh may also help to
prevent the absorbent material from moving around and/or exiting the suction chamber, and in some variations, may also help to prevent exudates collected in the suction chamber from exiting the chamber through the distal port or inlet.

Absorbent materials that may be used in a fluid retention assembly may be selected according to the expected viscosity (or other liquid characteristic) and/or quantity of the exudates. Certain absorbent materials may also be selected based on the desired absorption capacity. The absorption capacity of the material may be maintained under negative and/or positive pressure conditions. Some variations of an absorption material may hygroscopic, and may be able to absorb vapor. The fluid absorption material may be permeable to air, such that the negative pressure generated by the suction device may be conveyed to the wound without substantial hindrance. Suitable absorbent materials may be selected from natural, synthetic, and modified natural polymers and materials. Absorbent materials may be inorganic or organic materials, such as sodium acrylic-based polymers, silica gels, cross-linked polymers, etc. Other examples of absorbent materials may include gauze, pulp, sponges, foams, desiccated hydrogels, and cross-linked polyprotic resins. Suitable absorbent materials may be available from various commercial vendors, such as Dow Chemical Company located in Midland, Mich., U.S.A., and Stockhausen GmbH & Co. KG, D-47805 Krefeld, Federal Republic of Germany. Other examples of absorbent materials may include starch-acrylonitrile co-polymers, carboxy methyl cellulose (CMC), acrylic acid, polyvinyl alcohol (PVA) and isobutylene maleic anhydride (IMA), as well as various foams, including XTRASORB^{™}. Some variations of a fluid retention assembly may use a superabsorbent material, which may be capable of retaining an amount of water equal to at least 100% of its dry weight (e.g., as measured by the test of Intrinsic Absorbent Capacity). In some of the foregoing embodiments, the superabsorbent material may be Isolyser^{™} by Microtek Medical. Other examples of absorbent materials that may be used with a fluid retention assembly for a suction device may include sodium polyacrylate with sodium dichloro-S-triazinetrione dihydrate, cellulose based substrates, AQUA KEEP^{®} polymer products, etc.

In some variations, the fluid absorbent material may have a first non-hydrated state and a second hydrated state, where in the non-hydrated state the absorbent material may occupy a smaller volume than when in the hydrated state. For example, the absorbent material may expand as it absorbs fluids and transitions from the non-hydrated to hydrated configuration. In some variations, the absorbent material in the non-hydrated state may be powder-like, and in the hydrated state, the absorbent material may be gel-like, or may be a solid or a semi-solid. In other variations, the absorbent material may be a planar sheet or pad that thickens or expands as it absorbs fluid. The fluid absorbent material may be a porous material (e.g. a sponge, foam, textile, etc), and may be a planar or three dimensional porous matrix. An absorbent material that is a planar pad may have a first thickness in the non-hydrated state, and a second thickness in the hydrated state, where the second thickness is greater than the first thickness. Alternatively or additionally, the absorbent material may comprise loose components such as pellets, spheres, granules, clusters, powder, and the like. The particle sizes may range from about 20 µm to about 500 µm, for example, about 20 µm to 30 µm, or about 200 µm to 300 µm, or about 350 µm to 390 µm in the non-hydrated state. The absorbent material may also take the form of a collapsed woven material, such as a textile, or compressed polymer or sponge or porous matrix in its non-hydrated state. In the expanded hydrated state, the absorbent material may expand, and may be enlarged pellets or clusters, an expanded textile or sponge or porous matrix. In some cases, the absorbent material in the hydrated state may be a solid, a semi-solid, or a gel. Some variations of absorbent materials may decompose as it absorbs fluids. In some examples, the fluid absorbent material may be a volume neutral material, wherein the total volume of the separate fluid and separate absorbent material is approximately the same volume of the fluid and absorbent material when intermixed. For example, the separated total volumes and the intermixed volume may be equal, or at least within 5% or 10% of each other. In other examples, the fluid absorbent material may be a volume increasing material, wherein the intermixed volume is at least 15% or 25% or more than the total separated volumes.

Optionally, some variations of a fluid retention assembly may comprise a disinfectant, which may help to sanitize exudates that enter the collection chamber. For example, the disinfectant may be attached to, bonded to, embedded in, cross-linked with and/or otherwise incorporated with the absorbent material. In other examples, the disinfectant may be freely disposed within the collection chamber, or may be attached to other structures, such as the slidable seal assembly. The disinfectant may be anti-bacterial (e.g. bacteriostatic or bacteriocidal), anti-viral, anti-fungal, and/or anti-parasitic. Some examples of disinfectant compounds that may be used in a fluid retention system may include chlorhexidine, sodium hypochlorite, sodium dichloro-s-triazinetrione dehydrate (or other chlorine-based disinfectant), a sulfonamide, silver sulfadiazine, polyhexanide. In some variations, the absorbent material itself may also act as a disinfectant. For example, a fluid retention assembly may use a liquid medical waste solidifier, such as Isolyser LTS-Plus^{®} Solidifier or Isosorb^{®} Solidifier by Microtek Medical. Optionally, the fluid retention assembly may also comprise a deodorizer, such as zeolite, activated charcoal, silica gel, or hydrogen peroxide. In some variations, the disinfectant treat the collected exudates such that the expended device may be disposed as regular trash, rather than as biohazardous waste.

A fluid retention assembly may be installed in the suction chamber of a suction device in a variety of configurations. Fluid retention assemblies may comprise an absorbent material that may be sequestered in a portion of the suction chamber, temporarily or permanently. For example, a fluid retention assembly may comprise an absorbent pad or sheet that may be attached to the walls of the suction chamber so that it does not move within the suction chamber as the suction device changes orientation. Alternatively or additionally, a fluid retention assembly may comprise a screen (e.g., a mesh, filter, etc.) that may be attached at a distal portion of the suction chamber. For example, the screen may be attached within the distal portion of the suction chamber, just proximal to a distal portion leading to the distal port of the suction device. In some fluid retention assemblies, the absorbent material may be retained by a carrier structure, e.g. bonded to a surface of a supporting sheet or other structure, or enclosed in a pouch or other container. The pouch may freely move within the suction chamber, or may be attached to any desired region of suction chamber such that it remains at the desired region despite any changes in the orientation of the suction device. A fluid retention assembly may comprise a combination of one or more of the above described components, as may be desirable. For example, a fluid retention assembly may comprise absorbent materials enclosed in a pouch, where the pouch is sequestered to a portion of the suction chamber by one or more screens. A fluid retention assembly may comprise an absorbent pad or sheet that may be temporarily or permanently secured within the suction chamber using adhesives and/or one or more screens. Various examples of fluid retention assemblies are described below.

In some variations, the absorbent material of a fluid retention assembly may be retained by a carrier structure, such as a pouch. In some variations, the absorbent material may be enclosed in an internal pouch made of a semi-permeable membrane. This internal pouch may help to prevent the fluid absorption material from obstructing or clogging the various valve and/or conduits of the suction device. Other variations of fluid retention assemblies may comprise an absorbent material that has a self-contained form (e.g., a porous matrix, sponge, gauze, pad, foam, etc.). The absorbent material may be permeable to air, as may be desirable. In some examples, the absorbent material may be woven or non-woven sponges or gauze, and/or may be made of a porous material. In some variations, the absorbent material may be permeable to air, as may be desirable. The absorbent material may be made of any of the materials previously described. In some variations, the absorbent material may be retained by a carrier structure. For example, the absorbent material may be immobilized in a substrate (e.g., impregnated or woven into a matrix, adsorbed to a porous matrix, etc.). In some variations, the absorbent material may be bonded to the carrier structure and/or integrated with the substrate matrix. The absorbent material may or may not be sterile. Fluid retention assemblies comprising such absorbent materials may or may not include a screen or mesh to prevent movement of the absorbent material as the suction device changes orientation. An absorbent material, e.g., an absorbent pad, may be temporarily or permanently attached at any desirable portion of the suction device, for example, at a distal portion of the suction chamber. In some variations, the fluid retention assembly may be in the form of one or more super absorbent pads that remains generally intact without being enclosed in a pouch. Such pads may be attached to the inner walls of the collection chamber. The shape of the super absorbent pad may be selected to correspond with the cross-sectional shape of the suction device. For example, a suction device with an oval-shaped (e.g., non-circular) cross-section may have a corresponding oval-shaped super absorbent pad. The super absorbent pad may also comprise an opening therethrough that is configured to be aligned with the opening of the distal vacuum port such that it does not interfere with the transmission of negative pressure from the suction chamber to the wound. The opening may have a shape that may or may not correspond to the overall geometry of the pad, e.g., an oval-shaped pad may have an oval-shaped opening or a circle-shaped opening. The super absorbent pad may be attached to the distal wall of the collection chamber such that the pad does not shift proximally should the suction device be inverted and/or as the super absorbent pad retains liquids/solids. However, the attachment mechanism should still allow the super absorbent pad to expand as it takes up wound exudates. For example, the attachment mechanism may itself be flexible and/or expandable in accordance with the size of the super absorbent pad (which expands as it absorbs exudates). Some suction devices may have a fluid retention system that comprises two or more super absorbent pads. In such devices, the multiple super absorbent pads may be tethered together such that as one pad reaches saturation, absorption can continue to a second pad without a substantial gap (e.g., air gap) therebetween. The restraint mechanism that couples the multiple super absorbent pads together, as well as the attachment mechanism that couples the super absorbent pads to the walls of the collection chamber may allow for the expansion of the super absorbent pads as they take up wound exudates.

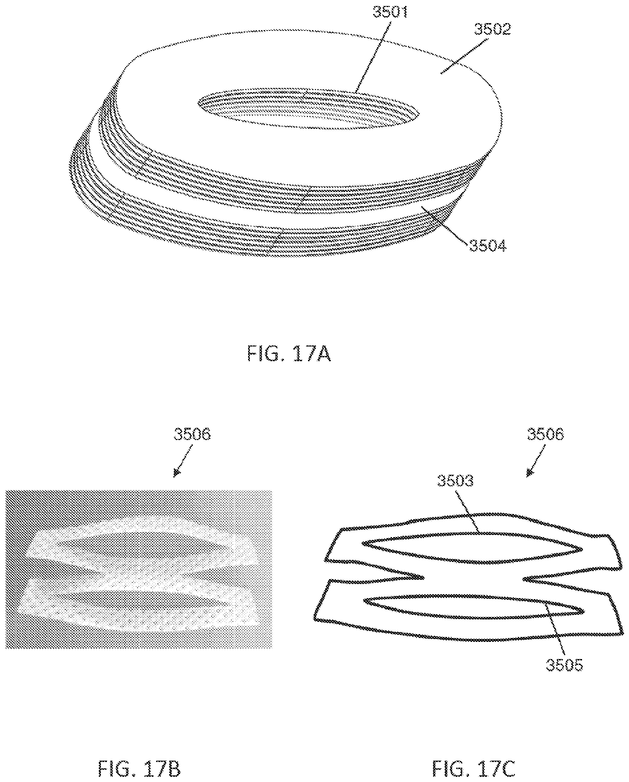

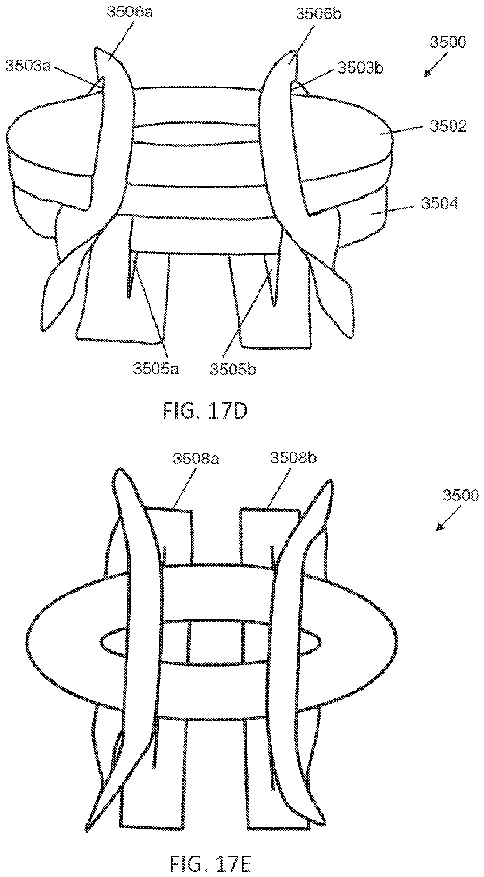

One example of a fluid retention system comprising one or more super absorbent pads is depicted in FIGS. 17A-E. The fluid retention system 3500 may comprise a first super absorbent pad 3502 and a second super absorbent pad 3504. Each of the super absorbent pads may have an opening 3501 therethrough. The opening 3501 may be circular or non-circular. The first and second super absorbent pads 3502, 3504 may be coupled to each other using a restraint mechanism or securing structure that includes one or more tethered loops (e.g., in a Figure-8 configuration), such as the looped structure 3506 depicted in FIGS. 17B-C. The restraint mechanism may also comprise at least one retention anchor, which may be a T-tag structure or a retention disc. The restraint mechanism may also retain the pads such that their apertures overlap with the distal conduit of the suction chamber. The super absorbent pads may be inserted into the openings 3503, 3505 of the looped structure 3506, which may in turn be attached to the wall of the collection chamber (e.g., the distal wall). FIG. 17D is a perspective side view of the fluid retention system 3500 that comprises the first and second super absorbent pads 3502, 3504 that are retained in the openings of a first tethered loop structure 3506a and a second tethered loop structure 3506b. The upper openings 3503a, 3503b of the tethered loop structures 3506a, 3506b may retain the first super absorbent pad 3502, while the lower openings 3505a, 3505b of the tethered loop structures 3506a, 3506b may retain the second super absorbent pad 3504. The bottom surfaces of each of the super absorbent pads 3502, 3504 may optionally have an adhesive, which may help to couple the super absorbent pads to each other and/or the wall of the collection chamber and/or to the tethered loop structures. The openings 3503a,b and 3505a,b may be sized such that the super absorbent pads can expand as they absorb exudates, but also control the degree of expansion and movement of the pad so that the pads do not float freely within the collection chamber. The tethered loop structures 3506 may be made of a thin (e.g., less than or equal to 0.015 inch), non-stretchable material. In some variations, the material may be stretchable, but to a limited degree so that the expansion and location of the super absorbent pads may be controlled. The material may be woven or non-woven, for example, 1 oz polypropylene that is spun bound and non-woven, nylon, mesh materials, polyethylene, acetate, organza, acrylic, silk, natural or synthetic materials, possibly coated to enhance performance characteristics, etc. In other variations, the restraint mechanism may comprise string or filament that weaves into and/or wraps around the super absorbent pads, and the ends of the string or filament may be attached (e.g., adhered) to the collection chamber wall. FIG. 17E depicts a top view of the fluid retention system 3500. A lower surface 3508a,b of each of the tethered loop structures 3506a,b may be adhered to the distal wall of the collection chamber. An adhesive-coated bottom surface of the super absorbent pad 3504 may also help to secure the fluid retention system 3500 to the distal wall of the collection chamber. Alternatively or additionally, a restraint mechanism may not be adhesively attached to the collection chamber, and may be mechanically attached to the collection chamber by a mechanical interfit.

It should be understood that while certain features are described in certain embodiments, such features may be included or excluded in various other embodiments.

Although the exemplary embodiments described above utilize one-way check valves configured to permit evacuation of the suction chamber, in other variations, other valves that are not specific to flow direction may also be used. For example, the one-way check valves located in the slidable seal described above may be substituted with normally closed valves that may be biased to the open position upon insertion of the push rod into the seal, or upon application of force against a valve opening mechanism by the push rod. In some other variations, the one-way check valves in direct communication with the suction chamber, e.g. the example in FIG. 14D, may be substituted with a normally closed valve that require user activation, e.g. push button or slide lever, to open as the suction chamber is reactivated.

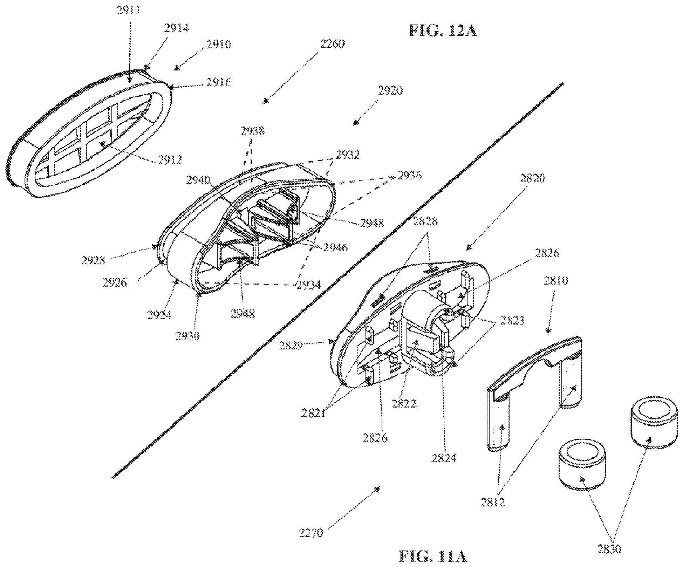

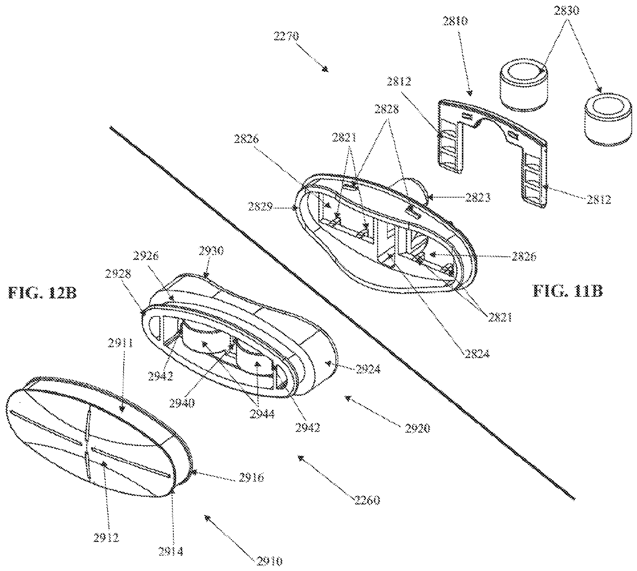

Turning to Figs. 11A and 11B now, the spring assembly 2270, which is mounted at the proximal end of the suction chamber and covered by the chamber rear cap, comprises a spring carrier 2820 and a U-shaped spring retainer 2810 containing two bushings 2830 mounted on the two vertical rails 2812 of the spring retainer 2810. Two substantially constant force springs (not shown in this figure) may each comprise a coiled body coupled to and wrapped around bushing 2830 and a free end distally extended and attached to the piston assembly. The springs may or may not be constant force springs. The spring attachment mechanism will be discussed in greater detail below. The spring carrier 2820 comprises a central opening 2824 and two side openings 2826. The central opening 2824 is configured to permit passage of the reset tool to access and displace the piston assembly. The side openings 2826 are configured to house the bushings 2830 and the springs when the spring retainer 2810 is coupled to the spring carrier 2820. As shown in this figure, multiple ridges 2821 may be located adjacent the side openings 2826 to limit the movement of the bushings 2830 and springs coiled around bushings 2830, thereby reducing deflections or deformations of the springs during operation of the suction apparatus. The spring carrier 2820 may also comprise resilient tabs 2822 that may slidably engage one or more grooves on the reset tool shaft, which may reduce angular deviations of the reset tool with respect to the longitudinal movement axis of the seal. The spring carrier 2820 may also comprises two interlocking structures 2823 configured to releasably lock the reset tool in place after the suction apparatus is charged. The interlocking mechanism will be described in detail later. Fixation structures 2828 may be provided to form a snap-fit or other type of interfit with complementary structures on the suction chamber.

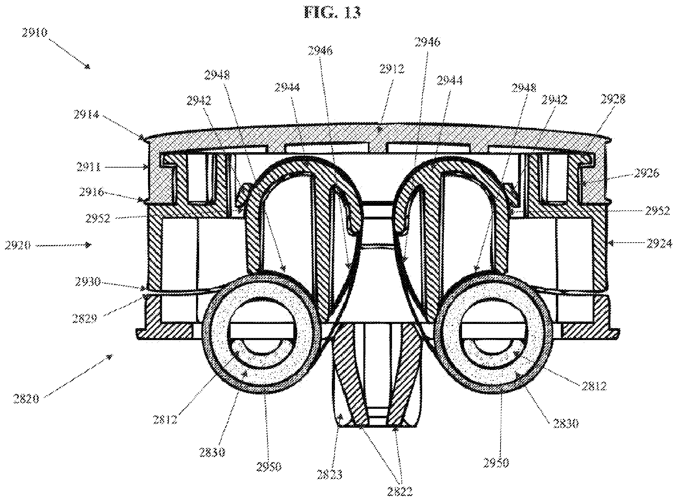

Figs. 12A and 12B are component views of the piston assembly 2260 that comprises a slidable seal 2910 and a piston 2920. The piston assembly 2260 may be configured to traverse between the distal end and the proximal end of the suction chamber while maintaining a substantially airtight seal. As mentioned previously, the piston assembly 2260 provides an airtight separation of the suction chamber between a collection chamber and a working chamber. In the depicted embodiment, the slidable seal 2910 has a non-circular, elliptical cross-sectional shape with respect to its movement axis in the suction chamber, but in other embodiments, other shapes as described herein may be used. The slidable seal 2910 may comprise a side wall 2911 and a distal end wall 2912. The side wall 2911 of the slidable seal 2910 further comprises a distal perimeter ridge 2914 and a proximal perimeter ridge 2916, the dimensions of which may be larger than that of the side wall 2911 of slidable seal 2910. The ridges 2914 and 2916 may be configured to be in a sliding contact with the interior surface of the suction chamber. They may provide a sealed contact while limiting sliding friction. The exterior surfaces of the slidable seal and/or the interior surfaces of the suction chamber may comprise a friction-reducing lubricant or a lubricious coating material. Although the slidable seal 2910 depicted in FIGS. 12A, 12B and 13 do not have a one-way valve, it should be understood that a one-way valve (such as any depicted and described above) may be incorporated in the slidable seal 2910.

The slidable seal 2910 may be detachably coupled to the piston 2920 or in some embodiments, the slidable seal 2910 and the piston 2910 may be integrally formed. In the depicted embodiment, the piston 2920 may comprise an elliptical frame with a side wall 2924. The distal portion of side wall 2920 may comprise a recess 2926 and a raised edge or flange 2928 configured form a complementary interfit with the slidable seal 2910. The proximal perimeter edge 2930 of side wall 2922 may have a complementary shape to the distal edge 2829 of the spring carrier 2820. In the depicted embodiment, both the proximal edge 2930 of the piston side wall 2922 and the distal perimeter edge 2829 of the spring carrier have a curved, non-planar configuration. As mentioned previously, the seal and/or seal mount (e.g. piston 2920) may have a variable longitudinal length along its perimeter. In some instances, an increased longitudinal dimension may provide additional stability to the seal along a dimension of the seal. In some examples, the side length along a section of the perimeter of the piston 2920 may be related to the transverse dimension intersecting a) that side length of the perimeter and b) the central movement axis of the seal and/or piston. In the example in Fig. 12A, the lateral longitudinal surface of the piston 2920 may have a longitudinal length 2932, based upon the increased width 2934 of the piston 2920 relative to the height 2936 of the suction chamber 2210 (corresponding to the increased width and reduced height of the suction chamber 2210). In comparison, the superior longitudinal surface of the piston 2920 may have a longitudinal length 2938 that is smaller than the longitudinal length 2932 of the lateral longitudinal surface from the reduced height 2936 of the piston 2920.

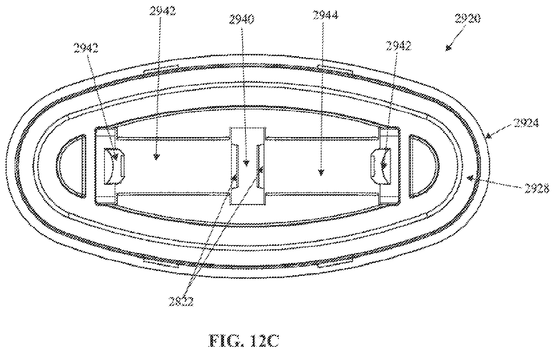

Referring to Figs. 12A, 12B and 13, the piston 2920 may also comprise a central opening 2940 which may be aligned with the central opening 2824 of spring carrier 2820. The piston central opening 2940 may be configured to provide passage of the distal ends of the constant force springs. Fig. 12C provides a frontal elevational view of the piston 2920. The distal regions 2952 of the constant force springs 2950 (depicted only in Fig. 13) may extend through the central opening 2940 and are coupled to a pair of spring retaining structures 2930 disposed on the front surface of piston 2920. In this particular embodiment, the retaining structures 2930 are configured to be inserted into apertures provided on the springs and may or may not maintain their coupling using residual spring force that may be present in the springs in the retracted configuration. The retaining structure and the springs may have any of a variety of other coupling configurations, however (e.g. the retaining structures may comprise posts which block displacement of T-shaped spring ends). Between the central opening 2940 and the retaining structures 2942 are curved support surfaces 2944 which are configured to push against the springs. In some examples, the length of the curved support surfaces 2944 between the central opening 2940 and the retaining structures 2930 may be at least one or one and a half times the width of the springs, while in other examples may be two or three times or four times the width of the springs. In some examples, the curved support surfaces 2944 provide a substantial surface area to distribute the pushing forces and may reduce the risk of damage to the springs. Referring back to Fig. 12A, the piston 2920 may further comprise convex supports 2946 adjacent to the central opening 2940, which may also support the springs as the springs converge into the central opening 2940. The convex supports 2946 may have a curved length of at least about the width of the springs, but in other examples may be at least two or three times the width of the springs. Referring to Figs. 12A and 13, the convex supports 2926 may also comprise a concave region 2948, which may accommodate the coils of the spring and the spring carriers 2830 when the piston assembly 2260 is in a retracted configuration. Although the piston assembly 2260 and the spring assembly 2270 depicted in Fig. 11A to 11B utilized two springs, in other examples, one spring, three springs, four springs, or five or more springs may be used. The number of springs, the type of springs, and the width and length of the springs may be varied, and in other examples, non-spring bias members may be used (e.g. sealed pneumatic shocks).

In some embodiments, the method of treating an area of damaged tissue may comprise affixing a sealant layer around an area of tissue to be treated; creating a sealed enclosure around the area of the tissue with the sealant layer, inserting a collection chamber into a housing chamber and charging the collection chamber; creating a fluid communication between the collection chamber and the sealed wound enclosure; activating the collection chamber to create a reduced pressure level within the sealed wound enclosure; if the collection chamber is filled up with wound exudates, terminating the fluid communication between the collection chamber and the wound seal and releasing the collection chamber from the wound site; withdrawing the collection chamber from the housing chamber and replacing it with a new collection chamber; and repeating the steps as appropriate to continue a reduced pressure treatment.

Although the embodiments herein have been described in relation to certain examples, various additional embodiments and alterations to the described examples are contemplated within the scope of the invention. Thus, no part of the foregoing description should be interpreted to limit the scope of the invention as set forth in the following claims. For all of the embodiments described above, the steps of the methods need not be performed sequentially. Accordingly, it is not intended that the invention be limited, except as by the appended claims.

## Claims

1. A system (101) for treatment of a patient, wherein the system is configured to be charged prior to applying reduced pressure, the system (101) comprising:
a. a suction chamber (202) with a vacuum conduit for delivering negative pressure to a wound, a rigid sidewall (3210) and a longitudinal axis, wherein the suction chamber (202) is configured to generate a vacuum in communication with the vacuum conduit;
b. a piston assembly (3234) comprising a slidable seal (3231) with a perimeter edge configured to slidably seal against the rigid sidewalls (3210) of the suction chamber (202) along the longitudinal axis;
c. a reset tool (3236) configured to displace the slidable seal (3231) into a charged position; and
d. a first one-way valve (3230) located across the slidable seal (3231), **characterised in that** the one-way valve (3230) is configured to be opened only when the reset tool (3236) is inserted into the system and pressed against the first one-way valve (3230) to permit fluids to flow from a distal portion of the suction chamber (202) to a proximal portion of the suction chamber (202), but not from the proximal portion of the suction chamber (202) to the distal portion of the suction chamber (202).

2. The system (101) of claim 1, further comprising a second one-way valve (3202) configured to permit entry of fluid into the suction chamber (202) and resist exit of fluid out of the suction chamber (202).

3. The system (101) of claim 2, wherein the second one-way valve (3202) is integrally formed along the vacuum conduit or wherein the second one-way valve (3202) is located in a connector (3200) configured to attach to the vacuum conduit or is located in the suction chamber (202) in communication with the vacuum conduit or is located outside of the suction chamber (202) in communication with the vacuum conduit.

4. The system (101) of claim 2, wherein the second one-way valve (3202) is located in a connector (3200) configured to attach to the vacuum conduit and wherein the connector (3200) has a proximal opening (3206) configured to connect with the suction chamber (202) and a distal opening (3204), wherein the second one-way valve (3202) is located between the proximal opening (3206) and the distal opening (3204) of the connector (3200).

5. The system (101) of any one of claims 1-4, further comprising a valve flap located over an opening of the first one-way valve (3230), wherein the valve flap is configured to permit the passage of air, but resists the passage of liquids or solids.

6. The system (101) of claim 5, wherein the valve flap is disposed over a stand-off over the first one-way valve (3230), wherein the stand-off comprises one or more gaps in communication with an opening of the first one-way valve (3230).

7. The system (101) of claim 6, wherein the valve flap comprises a first configuration where the valve flap does not obscure the one or more gaps of the stand-off and a second configuration where the valve flap obscures the one or more gaps.

8. The system (101) of any one of claims 6-7, further comprising an inner wall (3314) surrounding the opening of the first one-way valve (3230) and an outer wall (3316) surrounding the inner wall (3314), wherein the height of the inner wall (3314) is about equal to or greater than the height of the outer wall (3316).

9. The system (101) of claim 8, wherein in the first configuration, the valve flap does not contact the outer wall (3316) and in the second configuration, the valve flap contacts the outer wall (3316).

10. The system (101) of any one of claims 8-9, wherein the space between the inner wall (3314) and the outer wall (3316) defines a channel (3317).

11. The system (101) of any one of the above claims further comprising a filter (3306) located over the first (3230) and/or second (3202) valves.

12. The system (101) of any one of the above claims further comprising a first absorbent pad (3502) coupled to a distal wall of the suction chamber (202), wherein optionally the first absorbent pad (3502) comprises an opening (3501) and is coupled to the suction chamber (202) such that the opening (3501) is aligned with the vacuum conduit.

13. The system (101) of claim 12, further comprising a second absorbent pad (3504) and wherein the second absorbent pad (3504) comprises an opening (3501) and is coupled to the suction chamber (202) such that the opening (3501) is aligned with the opening (3501) of the first absorbent pad (3502).

14. The system (101) of any one of claims 12 to 13, wherein the first absorbent pad (3502) is coupled to the suction chamber (202) such that at least a portion of the first absorbent pad (3502) remains coupled to the distal wall of the suction chamber (202) after the absorbent pad (3502) has absorbed at least some fluid.

15. The system (101) of any one of claims 12 to 14, further comprising a restraint configured to attach at least one of the of first (3502) and second (3504) absorbent pads to the distal wall of the suction chamber (202), wherein optionally the restraint is further configured to maintain the first (3502) and second (3504) pads in contact together.

16. The system (101) of any one of claims 12 to 15, wherein at least one of the first (3502) and second (3504) absorbent pads comprises a super- absorbent material or wherein at least one of the first (3502) and second (3504) absorbent pads comprises an adhesive surface.

## Patentansprüche

1. Ein System (101) zur Behandlung eines Patienten, wobei das System konfiguriert ist, um vor der Anwendung von Unterdruck geladen zu werden, das System (101) aufweisend:
a. eine Saugkammer (202) mit einer Vakuumleitung zum Zuführen von Unterdruck zu einer Wunde, eine starre Seitenwand (3210) und eine Längsachse, wobei die Saugkammer (202) konfiguriert ist, um ein Vakuum in Verbindung mit der Vakuumleitung zu erzeugen;
b. eine Kolbenanordnung (3234), die eine verschiebbare Dichtung (3231) mit einer Umfangskante aufweist, die konfiguriert ist, um entlang der Längsachse verschiebbar gegen die starren Seitenwände (3210) der Saugkammer (202) abzudichten;
c. ein Rücksetzwerkzeug (3236), das konfiguriert ist, um die verschiebbare Dichtung (3231) in eine geladene Position zu verschieben; und
d. ein erstes Einwegventil (3230), das sich über der verschiebbaren Dichtung (3231) befindet, **dadurch gekennzeichnet, dass** das Einwegventil (3230) konfiguriert ist, um nur geöffnet zu werden, wenn das Rücksetzwerkzeug (3236) in das System eingeführt und gegen das erste Einwegventil (3230) gedrückt wird, um zu ermöglichen, dass Fluide von einem distalen Abschnitt der Saugkammer (202) zu einem proximalen Abschnitt der Saugkammer (202) fließen, aber nicht von dem proximalen Abschnitt der Saugkammer (202) zu dem distalen Abschnitt der Saugkammer (202).

2. Das System (101) nach Anspruch 1, ferner aufweisend ein zweites Einwegventil (3202), das konfiguriert ist, um einen Eintritt von Fluid in die Saugkammer (202) zu ermöglichen und dem Austritt von Fluid aus der Saugkammer (202) zu widerstehen.

3. Das System (101) nach Anspruch 2, wobei das zweite Einwegventil (3202) einstückig entlang der Vakuumleitung ausgebildet ist oder wobei sich das zweite Einwegventil (3202) in einem Verbinder (3200) befindet, der konfiguriert ist, um an der Vakuumleitung angebracht zu werden oder sich in der Saugkammer (202) in Verbindung mit der Vakuumleitung befindet oder sich außerhalb der Saugkammer (202) in Verbindung mit der Vakuumleitung befindet.

4. Das System (101) nach Anspruch 2, wobei sich das zweite Einwegventil (3202) in einem Verbinder (3200) befindet, der konfiguriert ist, um an der Vakuumleitung angebracht zu werden, und wobei der Verbinder (3200) eine proximale Öffnung (3206), die konfiguriert ist, um mit der Saugkammer (202) verbunden zu werden, und eine distale Öffnung (3204) aufweist, wobei sich das zweite Einwegventil (3202) zwischen der proximalen Öffnung (3206) und der distalen Öffnung (3204) des Verbinders (3200) befindet.

5. Das System (101) nach einem der Ansprüche 1 bis 4, ferner aufweisend eine Ventilklappe, die sich über einer Öffnung des ersten Einwegventils (3230) befindet, wobei die Ventilklappe konfiguriert ist, um den Durchgang von Luft zu ermöglichen, aber dem Durchgang von Flüssigkeiten oder Feststoffen widersteht.

6. Das System (101) nach Anspruch 5, wobei die Ventilklappe über einem Abstandshalter über dem ersten Einwegventil (3230) angeordnet ist, wobei der Abstandshalter einen oder mehrere Spalte in Verbindung mit einer Öffnung des ersten Einwegventils (3230) aufweist.

7. Das System (101) nach Anspruch 6, wobei die Ventilklappe eine erste Konfiguration aufweist, bei der die Ventilklappe den einen oder die mehreren Spalte des Abstandshalters nicht verdeckt, und eine zweite Konfiguration, bei der die Ventilklappe den einen oder die mehreren Spalte verdeckt.

8. Das System (101) nach einem der Ansprüche 6 bis 7, ferner aufweisend eine Innenwand (3314), die die Öffnung des ersten Einwegventils (3230) umgibt, und eine Außenwand (3316), die die Innenwand (3314) umgibt, wobei die Höhe der Innenwand (3314) etwa gleich oder größer als die Höhe der Außenwand (3316) ist.

9. Das System (101) nach Anspruch 8, wobei in der ersten Konfiguration die Ventilklappe die Außenwand (3316) nicht berührt und in der zweiten Konfiguration die Ventilklappe die Außenwand (3316) berührt.

10. Das System (101) nach einem der Ansprüche 8 bis 9, wobei der Raum zwischen der Innenwand (3314) und der Außenwand (3316) einen Kanal (3317) definiert.

11. Das System (101) nach einem der vorstehenden Ansprüche, ferner aufweisend einen Filter (3306), der sich über dem ersten (3230) und/oder zweiten (3202) Ventil befindet.

12. Das System (101) nach einem der vorstehenden Ansprüche, ferner aufweisend ein erstes absorbierendes Kissen (3502), das mit einer distalen Wand der Saugkammer (202) gekoppelt ist, wobei optional das erste absorbierende Kissen (3502) eine Öffnung (3501) aufweist und mit der Saugkammer (202) derart gekoppelt ist, dass die Öffnung (3501) mit der Vakuumleitung ausgerichtet ist.

13. Das System (101) nach Anspruch 12, ferner aufweisend ein zweites absorbierendes Kissen (3504) und wobei das zweite absorbierende Kissen (3504) eine Öffnung (3501) aufweist und mit der Saugkammer (202) derart gekoppelt ist, dass die Öffnung (3501) mit der Öffnung (3501) des ersten absorbierenden Kissens (3502) ausgerichtet ist.

14. Das System (101) nach einem der Ansprüche 12 bis 13, wobei das erste absorbierende Kissen (3502) mit der Saugkammer (202) derart gekoppelt ist, dass mindestens ein Abschnitt des ersten absorbierenden Kissens (3502) mit der distalen Wand der Saugkammer (202) gekoppelt bleibt, nachdem das absorbierende Kissen (3502) mindestens etwas Fluid absorbiert hat.

15. Das System (101) nach einem der Ansprüche 12 bis 14, ferner aufweisend eine Halterung, die konfiguriert ist, um mindestens eines des ersten (3502) und des zweiten (3504) absorbierenden Kissens an der distalen Wand der Saugkammer (202) zu befestigen, wobei optional die Halterung ferner konfiguriert ist, um das erste (3502) und das zweite (3504) Kissen in Kontakt miteinander zu halten.

16. Das System (101) nach einem der Ansprüche 12 bis 15, wobei mindestens eines des ersten (3502) und zweiten (3504) absorbierenden Kissens ein superabsorbierendes Material aufweist oder wobei mindestens eines des ersten (3502) und zweiten (3504) absorbierenden Kissens eine Klebefläche aufweist.

## Revendications

1. Système (101) pour le traitement d'un patient, dans lequel le système est configuré pour être chargé avant l'application d'une pression réduite, le système (101) comprenant :
a. une chambre d'aspiration (202) avec un conduit de vide pour délivrer une pression négative à une plaie, une paroi latérale rigide (3210) et un axe longitudinal, dans lequel la chambre d'aspiration (202) est configurée pour générer un vide en communication avec le conduit de vide ;
b. un ensemble piston (3234) comprenant un joint coulissant (3231) avec un bord périmétrique configuré pour sceller de manière coulissante contre les parois latérales rigides (3210) de la chambre d'aspiration (202) le long de l'axe longitudinal ;
c. un outil de réinitialisation (3236) configuré pour déplacer le joint coulissant (3231) dans une position chargée ; et
d. une première vanne unidirectionnelle (3230) située à travers le joint coulissant (3231), **caractérisé en ce que** la soupape unidirectionnelle (3230) est configurée pour être ouverte uniquement lorsque l'outil de réinitialisation (3236) est inséré dans le système et pressé contre la première soupape unidirectionnelle (3230) pour permettre à des fluides d'écoulement d'une partie distale de la chambre d'aspiration (202) vers une partie proximale de la chambre d'aspiration (202), mais pas de la partie proximale de la chambre d'aspiration (202) à la partie distale de la chambre d'aspiration (202).

2. Système (101) selon la revendication 1, comprenant en outre une seconde soupape unidirectionnelle (3202) configurée pour permettre l'entrée de fluide dans la chambre d'aspiration (202) et résister à la sortie de fluide hors de la chambre d'aspiration (202).

3. Système (101) selon la revendication 2, dans lequel la seconde soupape unidirectionnelle (3202) est formée d'un seul tenant le long du conduit à vide ou dans lequel la seconde soupape unidirectionnelle (3202) est située dans un connecteur (3200) configuré pour se fixer au conduit à vide ou est située dans la chambre d'aspiration (202) en communication avec le conduit à vide ou est située à l'extérieur de la chambre d'aspiration (202) en communication avec le conduit à vide.

4. Système (101) selon la revendication 2, dans lequel la seconde soupape unidirectionnelle (3202) est située dans un connecteur (3200 ) configuré pour se fixer au conduit de vide et dans lequel le connecteur (3200) a une ouverture proximale (3206) configurée pour se raccorder à la chambre d'aspiration (202) et une ouverture distale (3204), dans lequel la seconde soupape unidirectionnelle (3202) est située entre l'ouverture proximale (3206) et l'ouverture distale (3204) du connecteur (3200).

5. Système (101) selon l'une quelconque des revendications 1 à 4, comprenant en outre un clapet de soupape situé sur une ouverture de la première soupape (3230), dans lequel le volet de soupape est configuré pour permettre le passage d'air, mais résiste au passage de liquides ou de solides.

6. Système (101) selon la revendication 5, dans lequel le volet de soupape est disposé sur un retrait sur la première soupape unidirectionnelle (3230), dans lequel le retrait comprend un ou plusieurs espaces en communication avec une ouverture de la première soupape unidirectionnelle (3230).

7. Système (101) selon la revendication 6, dans lequel le volet de soupape comprend une première configuration où le volet de soupape n'obscurcit pas les un ou plusieurs espaces du retrait et une seconde configuration où le volet de soupape obscurcit les un ou plusieurs espaces.

8. Système (101) selon l'une quelconque des revendications 6 à 7, comprenant en outre une paroi intérieure (3314) entourant l'ouverture de la première soupape unidirectionnelle (3230) et une paroi extérieure (3316) entourant la paroi intérieure (3314), dans lequel la hauteur de la paroi intérieure (3314) est environ égale ou supérieure à la hauteur de la paroi extérieure (3316).

9. Système (101) selon la revendication 8, dans lequel dans la première configuration, le rabat de soupape ne vient pas en contact avec la paroi externe (3316) et dans la deuxième configuration, le rabat de soupape vient en contact avec la paroi externe (3316).

10. Système (101) selon l'une quelconque des revendications 8 à 9, dans lequel l'espace entre la paroi intérieure (3314) et la paroi extérieure (3316) définit un canal (3317).

11. Système (101) selon l'une quelconque des revendications précédentes, comprenant en outre un filtre (3306) situé sur les première (3230) et/ou seconde (3202) vannes.

12. Système (101) selon l'une quelconque des revendications précédentes, comprenant en outre un premier tampon absorbant (3502) couplé à une paroi distale de la chambre d'aspiration (202), dans lequel facultativement le premier tampon absorbant (3502) comprend une ouverture (3501) et est couplé à la chambre d'aspiration (202) de telle sorte que l'ouverture (3501) est alignée avec le conduit à vide.

13. Système (101) selon la revendication 12, comprenant en outre un second tampon absorbant (3504) et dans lequel le second tampon absorbant (3504) comprend une ouverture (3501) et est couplé à la chambre d'aspiration (202) de telle sorte que l'ouverture (3501) est alignée avec l'ouverture (3501) du premier tampon absorbant (3502).

14. Système (101) selon l'une quelconque des revendications 12 à 13, dans lequel le premier tampon absorbant (3502) est couplé à la chambre d'aspiration (202) de telle sorte qu'au moins une partie du premier tampon absorbant (3502) reste couplée à la paroi distale de la chambre d'aspiration (202) après que le tampon absorbant (3502) a absorbé au moins un certain fluide.

15. Système (101) selon l'une quelconque des revendications 12 à 14, comprenant en outre un dispositif de retenue configuré pour fixer au moins l'un des premier (3502) et second (3504) tampons absorbants à la paroi distale de la chambre d'aspiration (202), dans lequel facultativement le dispositif de retenue est en outre configuré pour maintenir les premier (3502) et second (3504) tampons en contact ensemble.

16. Système (101) selon l'une quelconque des revendications 12 à 15, dans lequel au moins l'un des premier (3502) et second (3504) tampons absorbants comprend un matériau super-absorbant ou dans lequel au moins l'un des premier (3502) et second (3504) tampons absorbants comprend une surface adhésive.
